(19) Europäisches Patentamt
European Patent Office
Office européen des brevets

(11) Publication number: 0 268 561 A2

(12) # EUROPEAN PATENT APPLICATION

(21) Application number: 87810671.5

(22) Date of filing: 16.11.87

(51) Int. Cl.4: **C 12 N 15/00**
C 12 P 21/02, A 61 K 37/02,
C 07 K 13/00

(30) Priority: 17.11.86 AT 3051/86 10.12.86 AT 3274/86
16.12.86 AT 3338/86 16.12.86 AT 3342/86
31.12.86 CH 5262/86
31.12.86 CH 5263/86
03.08.87 CH 2963/87
28.08.87 CH 3305/87

(43) Date of publication of application:
25.05.88 Bulletin 88/21

(84) Designated Contracting States:
AT BE CH DE ES FR GB GR IT LI LU NL SE

(71) Applicant: SANDOZ AG
Lichtstrasse 35
CH-4002 Basel (CH)

(84) Designated Contracting States:
BE CH ES FR GB GR IT LI LU NL SE

(71) Applicant: SANDOZ-PATENT-GMBH
Humboldtstrasse 3
D-7850 Lörrach (DE)

(84) Designated Contracting States: DE

(71) Applicant: SANDOZ-ERFINDUNGEN
Verwaltungsgesellschaft m.b.H.
Brunner Strasse 59
A-1235 Wien (AT)

(84) Designated Contracting States: AT

(72) Inventor: De Martin, Rainer
Hebelplatz 1
CH-4056 Basel (CH)

Fontana, Adriano Section of Clinical Immunology
Depart. of Internal Medicine University Hospital
Haldeliweg 4 CH-8044 Zürich (CH)

Hofer, Erhard
Rauracherstrasse 34
CH-4106 Therwil (CH)

Hofer-Warbinek, Renate
Rudolf Zeller Gasse 38/30
A-1238 Vienna (AT)

Wrann, Michael
Brunnerbergstrasse 31 62
A-2380 Perchtoldsdorf (AT)

The applicant has filed a statement in accordance with Rule 28 (4) EPC (issue of a sample only to an expert). Accession number(s) of the deposit(s): DSM 4225, DSM 4226, ECACC 87111101.

(54) **Production and use of a novel T-cell suppressor factor.**

(57) Human G-TsF has been isolated in substantially pure form. Nucleic acid encoding G-TsF and its precursor peptide has been isolated, cloned and expressed in several systems. G-TsF is recovered from transformed cultures for use in several therapeutic modalities.

EP 0 268 561 A2

**Description**

## PRODUCTION AND USE OF A NOVEL T-CELL SUPPRESSOR FACTOR

The present invention relates to the purification from natural sources of a T-cell suppressor protein, its preparation by recombinant methods and its use.

### 1. BACKGROUND

A new family of polypeptide factors that regulate cell growth and differentiation has emerged.

Three main lines of research may be distinguished which have led to factors related to the polypeptides of the present invention, namely the transforming growth factors β (TGF-βs), the cartilage-inducing factors (CIFs) and the glioblastoma-derived T-cell suppressor factor (G-TsF). The present invention is mainly concerned with G-TsF. As has become apparent after the priority dates which are claimed for the invention, G-TsF could be identical to TGF-β2 and/or CIF-B.

### 1.1. The TGF-βs

The TGF-βs are 25 kd disulfide-linked homodimers and heterodimers originally purified from human platelets. Other factors that are functionally and/or structurally related to the TGF-βs have also been found, e.g. new forms of TGF-β, activins, inhibins, and the Müllerian inhibiting substance (MIS) in mammals, as well as the product of the decapentaplegic gene complex in Drosophila.

These polypeptides are usually synthesized as part of larger secretory precursors as deduced for some of them from analysis of the corresponding cDNAs. The homologies of these proteins with each other reside mainly in the C-terminal domains of the full peptides, in the parts which are generally cleaved from the precursor to form the mature bioactive dimers. The conservation of multiple cysteines involved in intrachain and interchain disulfide bond formation is particularly striking. Common to several members of this family of polypeptides is their apparent involvement in differentiation processes such as embryogenesis and tissue repair.

Subsequently to the first priority date which is being claimed for the present invention it has become apparent that there are three forms of TGF-β, namely TGF-β1, TGF-β2 and TGF-β1.2, which result from homodimeric and heterodimeric combination of the subunits β1 and β2 (S. Cheifetz et al., Cell 48 [February 13, 1987] 409-415). The polypeptide originally described as TGF-β in fact consists of two β1 subunits. The β1 and β2 subunits are more closely related to each other than to any other known members of their gene family and exhibit about 70 % amino acid sequence similarity in their N-terminal halves.

Another striking aspect of the members of the TGF-β family which has only very recently emerged is the high degree of sequence conservation, e.g. 99 % between the human and mouse TGF-β1 sequences. This argues for a critical biological role of the TGF-βs across species.

The biological function of the TGF-βs has not been completely elucidated but as mentioned above is very varied. Thus in vitro they inhibit the proliferation of normal and certain tumor-derived epithelial cell lines, but induce proliferation of some mesenchymal cells as a possibly secondary response to elevated expression of autocrine mitogens. The expression of specific phenotypes by cells with differentiation potential is frequently profoundly altered by the TGF-βs. Thus they block adipogenesis, myogenesis and hematopoiesis while they promote chondrogenesis and epithelial cell differentiation in vitro, and can also modulate differentiated functions in lymphocytes, granulosa and adrenocortical cells.

The effects of the TGF-βs in vivo have been less thoroughly investigated but since they are present at relatively high levels in centers of active differentiation and in blood platelets an active involvement in the genesis of many types of tissue in normal development as well as in wound-healing responses is likely.

Indeed, many mesenchymal and epithelial cells whose differentiation and proliferation are affected by TGF-βs respond to these factors with elevated expression of fibronectin, various types of collagen and other cell-adhesion proteins. In vivo, subcutaneous administration induces abundant deposition of collagen.

Thus the TGF-βs appear to affect differentiation and morphogenesis by influencing or regulating the interaction of cells with the extracellular matrix, e.g. by controlling the abundance and architecture of the extracellular matrix as well as the ability of cells to interact with it, or by regulating receptors and intracellular targets for other growth factors.

The functions of TGF-β1 and TGF-β2 are not identical and they have different receptor-recognition properties. There may exist different receptors for TGF-β1 and TGF-β2, some of which are cross-reactive. This suggests some unique function for TGF-β2. Both factors seem, however, to be equipotent in inhibiting epithelial cell proliferation and adipogenesis and in induction of fibronectin and collagen expression.

The action of the TGF-βs is strongly anabolic, especially in connective tissue, and leads to fibrosis and angiogenesis.

Practical applications are thus in repair of tissue injury caused by trauma, burns, surgery, or debility in the aged, in osteoporosis; and, in view of their suppressive action on both T and B lymphocytes, as antiinflammatory or immunosuppressive agents.

The TGF-βs, particularly TGF-β1, might also be involved in the regulation of hematopoiesis (M. Ohta et al., Nature 329 [October 1987] 539-540).

The amino acid and nucleotide sequences for human TGF-β1 (originally named TGF-β) and its precursor are known from e.g. R. Derynck et al., Nature 316 (1985) 701-704; R. Derynck et al., J. Cell. Biochem. (1986) Suppl. 10 C, 105; R. Derynck et al., J. Biol. Chem. 261 (April 5, 1986) 4377-4379; and Genentech EP 200 341 (published on December 10, 1986).

TGF-β2 apparently had not been identified as such at the first priority of the present invention. Subsequently, partial amino acid sequences were published, e.g. for the first 29 N-terminal amino acids of mature porcine TGF-β2 in S. Cheifetz et al., Cell 48 (February 13, 1987) 409-415 and for the first 51 N-terminal amino acids of mature human TGF-β2 in T. Ikeda et al., Biochemistry 26 (May 1987) 2406-2410, the complete amino acid sequence of mature human TGF-β2 was then published in H. Marquardt et al., J. Biol. Chem. 262 (September 5, 1987) 12127-12131, i.e. later than the priority dates for the present invention.

1.2. CIF-B

Another line of research has been involved in the isolation, purification and characterization of the CIFs, i.a. of a bovine demineralized-bone protein named cartilage-inducing factor B (CIF-B), described e.g. in S.M. Seyedin et al., PNAS 82 (1985) 2267-2271, and various methods for extraction or partial purification of this or related factors are described e.g. in Collagen EP 121 976 and USP 4 627 982.

CIF-B is involved in endochondral bone formation since it is a potent inducer of chondrogenesis in vitro. In Collagen EP 182 483 compositions for implantation to effect bone repair are described, comprising a protein osteoinductive factor which might possibly include CIF-B. Collagen EP 213 776 discloses the use of i.a. CIF-B for treating inflammation or for treating a dysfunction or malfunction of hematopoiesis or lymphopoiesis.

Partial but inexact amino acid sequences for the 30 N-terminal amino acids of mature CIF-B isolated from bovine bone have been disclosed in e.g. Collagen EP 169 016 and, during the priority year for the present invention, in Collagen EP 213 776 (published on March 11, 1987); the correct amino acid sequence for the 30 first N-terminal amino acids of mature bovine CIF-B was published during the priority year for the present invention in S.M. Seyedin, J. Biol. Chem. 262 (February 15, 1987) 1946-1949.

It is now known that TGF-β2 and CIF-B are probably identical.

1.3. G-TsF

The third line of research has been concerned with glioblastoma-derived T-cell suppressor factor (G-TsF).

Patients with glioblastoma have depressed cell-mediated immunity. This was already apparent as early as 1972 (W.H. Brooks et al., J. Exp. Med. 136 [1972] 1631-1647). Both peripheral blood lymphocytes and the tumor-infiltrating lymphocytes of glioblastoma patients exhibit depressed in vitro proliferative responses. Furthermore the patients show impaired in vivo skin test reactivity to ubiquitous antigens. As an immunosuppressive activity is present in the tumor cyst fluid and serum before but not after removal of the tumor the defective cell-mediated immunity has been attributed to the production of immunosuppressive peptides by glioblastoma cells. In support of this conception is the observation that crude supernatants produced by several cultured tumor glioblastoma cell lines inhibit the lectin-induced thymocyte proliferation as well as the interleukin-2-induced growth of T cell clones and the generation of alloreactive cytotoxic T cells (A. Fontana et al., J. Immunol. 132 [1984] 1837-1844; M. Schwyzer and A. Fontana, J. Immunol. 134 [1985] 1003-1009).

G-TsF has been described in various states of purity in the literature and patented without sequence information in its natural, partially purified form e.g. as Sandoz EP 159 289, which describes the biochemical characterization and partial purification of the factor. Other publications are e.g. A. Fontana et al., J.Immunol. 132 [1984] 1837-1844 and M. Schwyzer and A. Fontana, J.Immunol. 134 [1985] 1003-1009.

The factor also increases the formation of connective tissue, has a strong anabolic activity and leads to fibrosis and angiogenesis.

During the priority year for the present invention, the purification of G-TsF to homogeneity and the amino-terminal sequence for the first 20 amino acids of the mature protein have been disclosed in e.g. M. Wrann et al., EMBO J. 6 (June 1987) 1633-1636.

2. SUMMARY OF THE INVENTION

From the foregoing it appears that there is a need for having the above factors isolated in pure form and further, in having them available in amounts large enough to enable preparation of derivatives, further testing and use in various therapeutic modalities, i.e. to have it available in recombinant form.

The present invention achieves this by a method comprising

a) isolating human G-TsF in substantially pure form from a natural source such as glioblastoma cells and

b) preparing recombinat G-TsF and its precursor peptide.

As regards a), this is achieved using techniques of protein chemistry. Two possible variants of G-TsF have been obtained, one of which was fully characterized.

The product G-TsF has a purity of at least 90%. Its specific activity is at least $5 \times 10^7$ units/mg in the thymocyte assay. Half-maximal inhibition is obtained at a concentration of $1 \times 10^{-11}$ M or less in that assay.

As regards b), the present invention makes available for the first time the complete amino acid and nucleotide sequences for mature G-TsF and G-TsF precursor and the corresponding cDNAs.

It thus provides recombinant mature G-TsF and a process for the preparation of mature G-TsF by recombinant DNA techniques, and corresponding cDNAs.

Further, it provides recombinant G-TsF precursor and a process for the preparation of G-TsF precursor by recombinant DNA techniques, and corresponding cDNAs.

Further, it provides recombinant G-TsF precursor part, and a process for the preparation of recombinant G-TsF precursor part by recombinant DNA techniques, and corresponding cDNAs.

The recombinant mature G-TsF or G-TsF precursor may be a human or a non-human, e.g. a mammalian form. Preferred is a human form.

The present invention thus makes possible for the first time the production of mature G-TsF, G-TsF precursor and G-TsF precursor part in amounts sufficient for therapeutic applications, further investigation of its multi-faceted biological activity and, based on knowledge of the sequences, the production of derivatives.

A preferred embodiment of cDNA and amino acid sequences for G-TsF is illustrated in fig. 2d. The full sequences are for G-TsF precursor. The arrow separates the sequences for the G-TsF precursor part from the sequences for mature G-TsF, the sequences for mature G-TsF also appear on fig. 2a.

As used herein, the expressions "precursor" and "precursor part" thus have different meanings, "precursor" covering the full peptide, including the mature peptide, whereas "precursor part" means the sequences for the full peptide minus the sequences for the mature peptide.

Allelic variants (i.e. naturally occurring base sequence changes which occur within a species which may or may not alter the amino acid sequence) of the nucleotide and corresponding peptide sequences of figs. 2a and 2d, and variations in the nucleotide sequence resulting from the degeneracy of the genetic code are also encompassed for use in the invention where they encode a polypeptide having G-TsF activity.

Variations in the sequences of figs. 2a and 2d which are caused by point mutations or larger modifications to enhance the activity or production of the protein should not, on expression, change the main functional and structural properties of the mature or precursor protein for which the sequence codes. Therefore, such variations in sequence are encompassed in the invention. Such nucleotide modifications deliberately engineered into the DNA sequence can be made by one skilled in the art using known techniques. Such modification can cause the deletion, insertion or substitution of amino acids in the peptide or prepeptide sequence for G-TsF. For example, the replacement of one or more of the cysteine residues in the coding sequence can eliminate a corresponding disulfide bridge. Additionally, the substitution, insertion or deletion of an amino acid at one or more of the tripeptide asparagine-linked glycosylation recognition sites in the precursor sequence can result in non-glycosylation at that site. Mutagenic techniques for such replacement or deletion are well-known.

The selection of suitable host cells and methods for transformation, culture, amplification, screening and product production and purification are known in the art.

Mammalian cells may be employed as host cells for production of G-TsF. One particularly suitable mammalian cell line is the Chinese Hamster Ovary (CHO) cell line. Another suitable mammalian cell line is the monkey COS-1 cell line, or the CV-1 cell line.

Yeast cells may also be used. Additionally, insect cells may also be utilized as host cells in combination with insect vectors, e.g. viral vectors, in the method of the present invention. Further, recombinant vaccinia viruses may be used to produce G-TsF in a broad variety of mammalian, e.g. human cells.

Another aspect of the present invention provides vectors for use in the method of expression of G-TsF which contain the same, or subtantially the same, nucleotide sequences as recited above. The vectors also contain appropriate control sequences permitting expression of the G-TsF DNA sequence. Alternatively, vectors incorporating modified or naturally occurring allelic sequences are also embodiments of the present invention. The vector may contain selected regulatory sequences in operative association with the above-described G-TsF DNA coding sequences which are capable of directing the replication and expression thereof in selected host cells. Useful regulatory sequences for such vectors are known in the art and may be selected depending upon the chosen host cells.

The method for preparing recombinant G-TsF comprises the recovery of expressed G-TsF from a host cell transformed with a gene coding for G-TsF. In particular, it comprises

a) construction of a vector which includes nucleic acid encoding G-TsF,

b) transformation of an heterologous host cell with the vector,

c) culture of the transformed host cell and

d) recovery of expressed G-TsF from the culture.

Nucleic acid that encodes mature G-TsF or G-TsF precursor is provided herein. It is useful in constructing the above vectors. This nucleic acid or a nucleic acid capable of hybridizing therewith may also be labelled and used in diagnostic assays for DNA or mRNA encoding G-TsF or its precursor or related proteins.

The preparation of G-TsF derivatives by recombinant methods is made possible by knowledge of the G-TsF coding sequence or the sequence coding for G-TsF and its precursor, disclosed herein. These derivatives include silent and expressed mutants in the nucleic acid encoding mature G-TsF or encoding G-TsF precursor.

Silent mutants involve the substitution of one degenerate codon for another where both codons code for the same amino acid but which substitution could exert a salutory effect on G-TsF yield in recombinant culture, e.g. by modifying the secondary structure of mRNA. Such a salutory substitution may be identified by screening G-TsF yields from transformants.

Expressed G-TsF mutants fall into one or more of three classes: deletions, substitutions or insertions. Deletions are characterized by the elimination of amino acid residues without the insertion of a replacement residue. Deletion-mutated G-TsF DNA is useful in making fragments, for example where it is desired to delete an immune epitope.

Substitution mutations are those in which one amino acid residue has been replaced by another. Such mutations are extremely difficult to make by methods other than recombinant synthesis, especially substitutions targeted for the interior of the primary amino acid sequence. They are useful in modifying the biological activity of G-TsF.

Insertional mutants are those in which one or more residues are placed into or at either end of the G-TsF nucleic acid. Fusions typically are a species of insertional mutants in which the insertion occurs at the carboxyl or amino terminal residues of G-TsF.

3. BRIEF DESCRIPTION OF THE DRAWINGS

Figure 1 shows the oligonucleotides used for screening the cDNA library. The two probes were based on the amino acid sequences shown. Position 22 was later shown to be isoleucine. Probe A consisted of two overlapping oligonucleotides, a 39-mer and a 42-mer, with 12 complementary nucleotides at their 3'-end. After annealing and filling-in with the Klenow fragment of DNA polymerase I, two 69-mers corresponding to the coding or noncoding strands were obtained. Positions of the predicted nucleotide sequence corresponding to the determined cDNA sequence are indicated by asterisks. Probe B was a mixture of sixteen 29-mers. At ambiguous positions deoxyinosine or deoxycytidine and deoxythymidine residues were inserted as indicated.

Figure 2a shows the complete nucleotide and amino acid sequences of mature human G-TsF. Amino acid similarities to TGF-β1 are underlined. The arrow indicates the protease cleavage site and the beginning of the mature protein.

Figure 2b depicts the sequencing strategy which was followed to arrive at the sequence of the cDNA in the region of the mature G-TsF. After subcloning of the EcoRI insert of lambda SUP25 in pBS.M13 part of the sequence was established by the Sanger dideoxy chain-termination method using Probe B (see Fig. 1) as primer. Two further oligonucleotides corresponding to parts of the obtained sequence were synthesized and employed to obtain the sequence coding for the major G-TsF peptide as depicted.

Figure 2c is a schematic diagram and partial restriction endonuclease map of G-TsF cDNA. The coding sequence is boxed and the mature peptide is indicated by the shaded part at the 3'-end. The sequenced cDNA inserts of lambdaSUP25, lambdaSUP40 and lambdaSUP 42 are aligned above the diagram.

Figure 2d shows the nucleotide and the amino acid sequences for the human G-TsF precursor. The nucleotide sequence of the region of lambdaSUP25 confirmed by two other independent clones (position 1-1695) is shown. A hydrophobic stretch of 15 amino acids probably constituting part of the pre-piece of the G-TsF precursor part and the mature G-TsF peptide at the carboxy-terminal end are underlined by a thick line. The postulated protease cleavage site at one end of the G-TsF peptide is indicated by an arrow. The stretch of basic amino acids preceeding the cleavage site is indicated by a dotted line. Three possible glycosylation sites in the precursor sequence are underlined with a thin line.

Figure 2e is a comparison of the amino acid sequences of G-TsF and TGF-β1 precursors analyzed according to the standard "align" program of Intelligenetics. Gaps are introduced to maximize the sequence similarities between the two proteins. Identical amino acids are indicated by asterisks. The 112 amino acids long mature forms of the peptides are boxed.

Figure 3a shows the CHO- and COS-cell expression vector named p91023(B)-SUP25-1 containing the full-length SUP25 cDNA. AdMLP = adenovirus major late promoter; DHFR = dihydrofolate reductase cDNA.

Figure 3b depicts the expression plasmid named pXMT3.neo7/SUP40-1 for CHO- and COS-cells with SUP40 cDNA. Ad MLP = adenovirus major late promoter; TKP = thymidine kinase promoter of Herpes simplex virus; DHFR = dihydrofolate reductase cDNA.

Figure 4 shows the final purification step for G-TsF from 308 glioblastoma cells from 1 l of glioblastoma supernatant on Pro-RPC® with trifluoroacetic acid/isopropanol. Fractions from reversed-phase FPLC on Pro-RPCTM were tested for inhibition of ConA-induced thymocyte proliferation at a final dilution of 1:5000. In addition, 100-microl aliquots of the fractions were lyophilized in the presence of 1 mg mannitol and analyzed by SDS-PAGE under reducing conditions on a 10-15 % polyacrylamide gel and silver staining. Lanes 29, 30 and 31 show the proteins present in the three fractions with G-TsF activity. The migration of the molecular weight marker proteins (BR) is indicated.

Figure 5 shows the activity of pure human G-TsF in two assay systems:

A. Dose-dependent inhibitory effect of G-TsF (●) and TGF-β1 (▲) on ConA-induced thymocyte proliferation. The solvent (0.1 % trifluoroacetic acid in 2-propanol 25 % v/v) used for elution of G-TsF from the final Pro-RPC® column was taken as a buffer control for G-TsF.

B. Suppression of IL-2-dependent T cell growth by G-TsF and TGF-β1. Ovalbumin-specific T helper cells were cultured in the presence of various concentrations of recombinant IL-2 together with G-TsF (●) at a concentration of 1.6 × $10^{-11}$ M or TGF-β1 (▲) at a concentration of $10^{-10}$ M. Controls consisted of solvents (see above) or medium in which TGF-β1 was diluted (o/Δ).

Figure 6 shows the final purification of G-TsF from 10 l of glioblastoma supernatant on Pro-RPC® with trifluoroacetic acid/isopropanol. Fractions from reversed-phase FPLC on Pro-RPC® were tested in the thymocyte proliferation assay. The insert shows the SDS-PAGE of 100-microl aliquots from fractions 28 to 34 as described for Fig. 4.

Figure 7a is a map of the plasmid named pP$_L$S1-SUP25-1 for expression of G-TsF in E.coli based on the lambda promoter. $P_L$ = lambda promoter.

Figure 7b shows the construction of the expression plasmid pP$_L$S1-SUP25-1. The partial HaeIII-XbaI fragment of G-TsF cDNA was derived by first converting the HaeIII site at position 2141 of the lambdaSUP25 cDNA with XbaI linkers to an XbaI site. Then partial cleavage of the cDNA with HaeIII and XbaI was performed.

Figure 8 shows the expression and purification of recombinant G-TsF from E.coli:

A. Immunoblot of total E. coli protein recovered following induction of the lambda promoter $P_L$ at 42° C. Lanes 0 to 6 are for the various incubation times.

B. SDS-PAGE of fractions 30 and 31 from the reversed-phase FPLC on Pro-RPC® showing that essentially pure G-TsF is recovered from Pro-RPC®

Figure 9 indicates the inhibition of thymocyte proliferation by recombinant G-TsF from E. coli.

Figure 10shows the demonstration of expression of recombinant G-TsF in an immunoblot of cell supernatants obtained from CHO cells.

Lane 1 = supernatant from control CHO cells transfected with the vector devoid of cDNA (pXMT3.neo7).

Lane 2 = supernatant from CHO cells transfected with vector containing G-TsF cDNA (pXMT3.neo7/SUP40-1) and selected at 1 nM methotrexate.

Lane 3 = as Lane 2, but selected at 10 nM methotrexate.

Figure 11 shows the inhibition of thymocyte proliferation with recombinant G-TsF from CHO cells. Dilution = final dilution of CHO supernatant in thymocytes assay.

□ = supernatant from control CHO cells transfected with pXMT3.neo7, without acid activation.

O = as □, but with activation with 1 M acetic acid overnight at room temperature.

■ = supernatant from CHO cells transfected with pXMT3.neo7/SUP40-1 and selected at 10 nM methotrexate, without acid activation.

● = as ■, but with acid activation as for O.

Some endogenous inhibition by acid-activated CHO cells is visible, which could be caused by G-TsF or some other factor such as TGF-β1 produced by the CHO cells; however, the production of recombinant material gives rise to an increase in suppressive activity of at least 2 orders of magnitude.

## 4. DETAILED DESCRIPTION

4.1. In a first aspect the G-TsF was purified to homogeneity and the first 20 amino acids of the mature human protein sequenced:

G-TsF was purified, after concentration and diafiltration of glioblastoma cell-derived supernatants, in a process including chromatography on hydroxylapatite and Pro-RPC®, cation-exchange chromatography on Mono-S®and a final reversed-phase FPLC on Pro-RPC®. The decisive step in the purification scheme was the cation-exchange chromatography on Mono-S®which resulted in a 16-fold purification and allowed the correlation of the biological activity to a 12.5 kd protein band on SDS-PAGE. This was confirmed by the final reversed-phase chromatography step (see Fig. 4) since after SDS-PAGE and silver staining a single band of 12.5 kd was detected in three fractions. The intensities of these bands correlated with G-TsF activity in the thymocyte assay. Moreover, the specific activity of 5 × $10^7$ units/mg calculated for the 12.5 kd band is comparable to other cytokines.

From this material the first 20 amino-terminal amino acids of mature G-TsF (see also Fig. 2a) were obtained by gas-phase sequencing of the peak fraction: AlaLeuAspAlaAlaTyrCysPheArgAsnValGln
5
AspAsnCysCysLeuArgProLeu .
15 20

As demonstrated in Fig. 2e both human G-TsF and TGF-β1 from porcine platelets(R&D Systems) inhibit the Concanavalin-A-induced proliferative response of thymocytes. The concentration required for half-maximal inhibition of thymocyte activation is $10^{-11}$ M for TGF-β1. An almost identical dose-response curve is observed by using purified G-TsF instead of TGF-β1, the half-maximal inhibition occurring at 8 × $10^{-12}$ M. Since crude supernatant of 308 glioblastoma cells was originally found to suppress T cell activation by interfering with the effect of IL-2, purified G-TsF and TGF-β1 were analyzed on an ovalbumin-specific T helper cell line (OVA-7T) established from C57BL/6 mice. At the concentrations tested (G-TsF at 1.6 × $10^{-11}$ M; TGF-β1 at $10^{-10}$ M) both factors inhibited the IL-2-dependent growth of OVA-7 T cells almost completely (Figure 5, part B). The degree of inhibition was independent of concentrations of IL-2 between 1 and 256 units/ml.

In its biologically active form the human G-TsF is a dimer of two identical subunits joined by interchain cross-linking over disulfide bridges. Dimerization may precede proteolytic processing. Heterodimers with TGF-β1 are also biologically active.

Biologically active G-TsF is herein defined as G-TsF which is capable of inhibiting Concanavalin-A-induced thymocyte proliferation and IL-2-induced growth of T-cell clones. These assays are described in the Example hereunder and various publications. This also defines the meaning of the expression "G-TsF-like activity".

"Mature G-TsF" when used without further qualification means a protein having more than 71 % amino acid sequence similarity with the amino acid sequence indicated in Fig. 2d after the arrow, e.g. ≧90 %.

"G-TsF precursor" when used withoug further qualification means a protein having more than 30 % amino acid sequence similarity with the full amino acid sequence indicated in Fig. 2d, e.g.≧60 %.

In one aspect the present invention thus provides for substantially pure human G-TsF, and for a process for the preparation of substantially pure human G-TsF comprising a cation-exchange chromatography step, preferably a cation-exchange chromatography step over Mono-S®. In particular, the process comprises

a) concentration and diafiltration of supernatants from cells from a natural source of G-TsF such as glioblastoma cells,

b) chromatography over hydroxylapatite,

c) reversed-phase chromatography over Pro-RPC®using trifluoroacetic acid/acetonitrile (2x) as an eluant,

d) cation-exchange chromatography, preferably over Mono-S®, and

e) reversed-phase chromatography over Pro-RPC®using trifluoroacetic acid/2-propanol as an eluent.

A further purification of G-TsF from 10 l of glioblastoma supernatants yielded a broad double-peak of the 12.5 kb material during the last reversed-phase FPLC on Pro-RPC®, which indicated the presence of two overlapping peaks of two slightly separated peptides. Material from the whole area of the double-peak showed a similar bioactivity in the thymocyte proliferation assay (see Fig. 6). Therefore, material from the first and second peak was subjected to gas-phase sequencing. Material from the first peak (fraction 30) gave an aminoterminal sequence identical to the one shown in Fig. 2a. However, material recovered from the second peak (fraction 32) yielded the following aminoterminal sequence:

```
AlaLeuAspAlaAlaTyrCysPheArgAsnValGlnAspAsn
 1       5          10
CysIleLeuArgProLeu
15          20          TyrIleBlankPheVal
                                       25
```

This partial sequence differs from the corresponding sequence in Fig. 2a in having Ile instead of Cys in position 16 and Val instead of Lys in position 25 (the amino acid corresponding to the Asp in position 23 of the material from fraction 30 could not be determined with certainty).

This further sequence could well be that for the N-terminal portion of a variant of human G-TsF. The present invention also encompasses this and further variants.

4.2. In a second aspect the above sequence information was then used to produce G-TsF by recombinant DNA techniques.

4.2.1. This production may be effected in accordance with generally known methods, e.g. using procedures similar to those described in T. Maniatis et al., Molecular Cloning, A Laboratory Manual, Cold Spring Harbor Laboratory, New York (1982).

However, as for TGF-β1, recombinant G-TsF has proven difficult to synthesize while retaining its activity. As can be seen from Fig. 2a the mature amino acid sequence contains a large number of cysteine residues, at least some of which apparently are involved in interchain crosslinking in forming the homodimeric G-TsF which is recovered from natural sources. Furthermore, G-TsF is expressed as a precursor molecule having a large amino terminal region which probably is involved in directing the later fate of G-TsF, i.e. transport across the endoplasmatic reticulum membranes, proper folding of the precursor polypeptide including formation of inter- and intramolecular disulfide bridges, storage in secreting vesicles and regulation of proteolytic cleavage followed by secretion of the bioactive peptide. However, it has been possible to transform eucaryotic cells to express heterologous G TsF, notwithstanding the difficulty in properly processing the primary translation product in recombinant culture. Production of mature G-TsF peptide in E. coli was also accomplished.

The invention encompasses recombinant mature G-TsF and its precursor part, their recombinant synthesis and the formation of bioactive G-TsF via its precursor; particularly, biologically active G-TsF having the sequences of Fig. 2d,including precursor G-TsF, mature G-TsF and G-TsF precursor part, and polypeptide fragments, — — — — — insertion, substitution and/or deletion mutants thereof.It comprises for example the preparation of bioactive dimeric G-TsF via the prior synthesis of the G-TsF precursor or of parts of the G-TsF precursor essential in folding and proteolytic processing.

The oligonucleotide probes shown in Fig. 1 were deduced from the G-TsF partial protein sequence and were used to screen a cDNA library derived from the human glioblastoma cell line 308. This library established in lambda gt10 has previously been size-selected for inserts larger than 1 600 bp. Upon screening a total of 100 000 recombinant phages a single clone was detected giving a signal with both probes. By using probe - - - B as a sequencing primer it was found that it contained sequences homologous to TGF-β1. This clone, dubbed lambda-SUP25, was subsequently used to isolate additional cDNA clones for G-TsF (lambdaSUP40 and lambda SUP42).

The sequencing strategy followed to establish the nucleotide sequence in the region coding for the mature peptide is shown in Fig. 2b.

A partial restriction enzyme map of the G-TsF cDNA is shown in Fig. 2c. The nucleotide sequence as determined by sequencing both strands of the insert in clone lambdaSUP25 from position 1 to 1695 and the deduced amino acid sequence, including that of the precursor protein , are shown in Fig. 2d. Sequencing of the inserts of clones lambda SUP40 and lambda SUP42 was carried out for 300 nucleotides at both ends and for the whole region coding for the mature G-TsF peptide with the help of specific internal oligonucleotide primers. These cDNAs start within several nucleotides from the 5′ end of lambda SUP25 and terminate at positions 1534 and 1695, respectively. Otherwise their sequences were found to be identical to that of the lambda SUP25 insert, which however is longer at the 3′ end.

The 5′-untranslated region of the G-TsF cDNA is AT rich (71 % AT) and an ATG translation initiation codon is located at positions 182-184. This codon is most likely used for initiation of translation because it is the first ATG in an open reading frame coding for a 414 amino acid - long protein containing the amino-terminal sequence of the G-TsF peptide at amino acid positions 303-323. In addition it is immediately preceeded by several stop codons in the same reading frame and the first 20 amino acids of the predicted protein show the strong hydrophobicity characteristic of signal peptides. The first cluster of significant homology to the predicted TGF-β1 precursor occurs from amino acid 21 to 24. No homology to the consensus sequence observed around the translation initiation site of other mRNAs is found except a purine at position -3. However, the following ATG codons at positions 266, 278 and 503 are not followed by a sequence coding for a significant run of hydrophobic amino acids.

Post-translational cleavage of the precursor gives rise to the mature G-TsF. The disposition of the precursor part is not known but may give rise to other biologically active peptides. The G-TsF precursor contains several pairs of basic residues which could also undergo post-translational cleavage and give rise to separate polypeptide entities.

4.2.2. Construction of a cDNA library

RNA was isolated using the urea-LiCl method and polyadenylated RNA was selected by oligo(dT) cellulose chromatography. The first cDNA strand was then synthesized with reverse transcriptase and the RNA/DNA hybrid converted to double-stranded cDNA. The cDNA was made blunt-ended with S1 nuclease and T4 DNA polymerase, methylated with EcoRI methylase, ligated to phosphorylated EcoRI linkers and the products digested with excess amounts of EcoRI. The cDNA was size-selected and fragments over 1 600 bp recovered by phenol extraction and ethanol precipitation. After ligation to lambda gt 10 arms the recombinant DNA was incubated with an in vitro packaging extract and the library established following infection of E. coli C600 hfl by plating on BBL agar plates and eluting the phages from the top-agar layer.

4.2.3. Screening of the library

Approximately 100 000 recombinant phages were plated in E.coli C600 and the plaques replicated onto nylon filters. The filters were then processed and screened using two probes derived from the assumed amino-terminal amino acids of G-TsF (Fig. 1). The oligonucleotide probes were synthesized with a DNA Synthesizer. One was a mixture of sixteen 29-mer corresponding to a ten amino acids - long portion. It was labelled with T4 polynucleotide kinase and [gamma-$^{32}$p]ATP. The second probe consisted of a 39-mer and a 42-mer (Fig. 1) with 12 complementary bases at their 3′-end, which could therefore be filled-in after annealing using [α-$^{32}$P]dGTP and the Klenow fragment of DNA polymerase I. Filters were prehybridized for 4 to 6 hours and then hybridized with the probes for 16 hours at 42°C. The filters were washed at 42°C in 5× NET, 0.2 % SDS and then again at gradually increasing temperatures up to 55°C. Areas of plaques giving signals with both probes were replated and rescreened at 47°C. A single plaque was found to give reproducible signals with both probes and the cDNA recovered from it was later used to rescreen the library.

4.2.4. Subcloning and sequencing

Lambda DNA was extracted from the positive clones following a minipreparation procedure. The cDNA inserts were recovered after EcoRI cleavage and subcloned into the pBS.M13 sequencing vector (Stratagene). DNA sequencing was performed using the dideoxy chain-termination method of Sanger. To obtain an overlapping set of sequences in both directions, the insert was first digested with HindIII. Subclones were then obtained from the HindIII fragments either by exonuclease and mung bean nuclease digestions or by cleavage at restriction sites revealed in partial sequence data. Additional sequence information was obtained in the region of the mature G-TsF peptide with the help of synthetic oligonucleotide primers.Ambiguous areas remaining after sequencing the whole region in both directions with Klenow DNA polymerase were resolved using reverse transcriptase for the chain elongation reaction.

The sequencing (see Fig. 2d) showed that G-TsF is translated as a precursor protein from which the mature protein has to be released by proteolytic cleavage. This active protein is a dimer of two mature peptide parts. The amino acid sequence of the mature G-TsF peptide starts at amino acid position 303 of the precursor. The G-TsF precursor contains three potential N-glycosylation sites.

The calculated molecular weight for the mature G-TsF of Fig. 2d is 12 320 daltons. The full molecule, including both the precursor part and the mature part, has a calculated molecular weight of 45 540 daltons (disregarding a possible glycosylation).

Mature G-TsF may be O-glycosylated and G-TsF precursor may be O- and/or N-glycosylated. Such glycosylated forms are also part of the invention.

Allelic and/or variant forms are also part of the invention.

Comparison of the sequence of mature G-TsF with sequences published for related factors during the priority year for the present invention, namely for the first 29 N-terminal amino acids of porcine TGF-β2 (S. Cheifetz et al., Cell 48 [February 13, 1987] 409-415) or for the first 51 N-terminal amino acids of human TGF-β2 (T. Ikeda et al., Biochemistry 26 [May 5, 1987] 2406-2410), or for the first 30 N-terminal amino acids of bovine CIF-B (S.M. Seyedin et al., J. Biol. Chem. 262 [February 15, 1987] 1946-1949) shows complete identity: thus G-TsF is the human analogue of the above factors and the considerable structural similarity confirms the very strong interspecies sequence conservation which is also observed in the case of TGF-β1.

As already mentioned above under 1., the complete amino acid sequence of mature human TGF-β2 has been published independently during the priority year for the present invention (H. Marquardt et al., J. Biol. Chem. 262 [September 5, 1987] 12127-12131); that sequence is identical to the amino acid sequence for mature G-TsF in Fig. 2d.

G-TsF shows further an amino acid sequence similarity of 71 % with TGF-β1. The similarity is extremely high from amino acid 329 to 346 and for the 14 C-terminal amino acids (Fig. 2e). This is also seen with the two more distantly related peptides inhibin and Muellerian inhibiting substance where the corresponding regions are the most conserved parts of the molecules. The structural similarity is further strengthened by the conservation of all nine cysteine residues in G-TsF. The amino acid sequence similarity of G-TsF precursor with TGF-β1 precursor is 30 %.

Some structural patterns are also conserved in the precursor parts of TGF-β1 and G-TsF. The mature peptides are obviously released by proteolytic cleavage at Lys-Arg residues. The structural homology between the G-TsF and TGF-β1 precursors is far less pronounced than between the mature proteins but there are definite clusters of sequence homology scattered over the precursor part. This indicates a long evolutionary divergence, a possibly different regulation in the production of the factor and possibly a modified biological role. The conserved sequence clusters may constitute functional domains of the precursor molecule involved in the transport and processing of the molecule and in the control of the release of the G-TsF peptide.

Characteristics such as molecular weight and the like for the native or wild type mature G-TsF of Fig. 2d obtained from glioblastoma cells are descriptive only for the native species of G-TsF. The mutants contemplated herein may vary the characteristics of native G-TsF considerably, and this in fact may be the objective of the mutagenesis. While G-TsF as defined herein includes native G-TsF, other related biologically active polypeptides will fall within the definition. G-TsF species like insertion mutants, deletion mutants, or fusion proteins will bring the mutant outside of the molecular weight established for native G-TsF. For example, fusion proteins with mature G-TsF or G-TsF precursor will have a greater molecular weight than native, mature G-TsF or G-TsF precursor, while deletion mutants of mature G-TsF or G-TsF precursor will have a lower molecular weight. Similarly, G-TsF or G-TsF precursor may be engineered in order to introduce glycosylation sites or to substitute serine for cysteine at sites not critical for biological activity. Further, post-translational processing of human pre-G-TsF, e.g. in cell lines derived from non-primate mammals, may produce micro-heterogeneity in the amino terminal region of mature G-TsF, so that alanine will no longer be the amino-terminal amino acid.

Further precursors may be fusion proteins in which mature G-TsF is linked by a peptide bond at its carboxyl terminus to an insoluble or gelatinous protein. The sequence at or within the region of this peptide bond is selected so as to be susceptible to proteolytic hydrolysis whereby G-TsF is released, either in vivo for in situ generation or, as part of a manufacturing protocoll, in vitro.

Recombinant G-TsF ordinarily means mammalian, preferably human, recombinant G-TsF; recombinant G-TsF from sources such as murine, porcine, equine or bovine is however included within the definition of recombinant G-TsF so long as it otherwise meets the standard described above for biological activity. G-TsF is not species-specific. Therefore, G-TsF from one species may be used in therapy of another species.

DNA which encodes G-TsF may be obtained by chemical synthesis, by screening reverse transcripts of mRNA from glioblastoma or other cells or by screening genomic libraries from eucaryotic cells. This DNA need not use the codons set forth in Fig. 2d so long as the host cell recognizes the codons which are used, and indeed, codon optimization according to the expression system used may lead to improved production.

4.3. <u>Expression</u> may be obtained in any suitable pro- or eucaryotic expression system using known methods, e.g. as described in Genentech <u>EP 200 341</u>.

4.3.1. Suitable expression vectors are DNA sequences encoding G-TsF and operably linked to suitable control sequences capable of effecting the expression of G-TsF in the host. Such control sequences include a transcriptional promoter, an optional operator sequence to control transcription, a sequence encoding suitable mRNA ribosomal binding sites, and sequences which control termination of transcription and translation. For expression of G-TsF in eucaryotic cells the vector also should include DNA encoding a selection gene. The selection gene can be supplied by an unlinked plasmid in cotransformation.

Vectors comprise plasmids, viruses (including phage), and integratable DNA fragments, i.e. fragments that are integratable into the host genome by recombination.

DNA regions are operably linked when they are functionally related to each other. For example DNA for a presequence or secretory leader is operably linked to DNA for a polypeptide if it is expressed as a preprotein which participates in the secretion of the polypeptide. A promoter is operably linked to a coding sequence if it controls the transcription of the sequence. A ribosome binding site is operably linked to a coding sequence if it is positioned so as to permit translation. Generally, operably linked means contiguous and, in the case of secretory leaders, contiguous and in reading phase.

Preferred host cells are cells derived from multicellular organisms. In principle any higher eucaryotic cell culture is workable, whether from vertebrate or invertebrate culture. Examples of useful host cell lines are Chinese Hamster Ovary (CHO) cell lines and COS-7 cell lines.

G-TsF is recovered from lysed, transformed cells and soluble cell debris separated by centrifugation. Alternatively, the culture supernatants from transformed cells that secrete G-TsF are simply separated from the cells by centrifugation. Then the G-TsF generally is purified by methods known in the art, e.g. using gel filtration in the presence of acid followed by HPLC and elution on an acetonitrile gradient.

As a further or substitute purification step cell lysates or supernatants are heated for a period and at a temperature sufficient to denature and precipitate contaminant proteins but not G-TsF. As TGF-β1, G-TsF is a remarkably heat-stable protein, perhaps as a result of extensive disulfide bond formation. As a result the heating should be conducted in a medium that contains low amounts of disulfide reagents such as dithiothreitol. Heating may also be combined with acidification since G-TsF is stable to 1M acetic acid.

Mature G-TsF is not glycosylated. Therefore it is separated from any residual contaminant heat-stable and acid-stable glycoproteins by adsorbing the glycoproteins on lectin columns such as lentil lectin-linked sepharose.

If high purity product is desired the crude or partially purified mixture is subjected thereafter to chromatofocusing.

Similar methods may be used to purify the precursor peptide.

4.3.2. Expression in CHO cells

In one embodiment expression was effected in CHO cells, in two variants:

4.3.2.1. First variant: with p91023(B)-SUP25-1

The full-length SUP25 cDNA was cloned into the CHO and COS-cell expression vector p91023B (G.G. Wong et al., Science 228 [1985] 810-815) and a clone having the correct orientation selected by restriction enzyme analysis (see Fig. 3a). The obtained plasmid DNA was then transfected into the dihydrofolatered-uct e-deficient CHO cell line DUKX-B - - - - - - by electroporation. All clones having incorporated the p91023(B)-SUP25-1 DNA were then selected in α(-)-medium under conditions where only cells producing dihydrofolate reductase will survive. Since the vector p91023(B) provides a gene for mouse dihydrofolate reductase transfected cell clones can be selected. By further selection employing the drug methotrexate in increasing concentrations starting with 1 nM the transfected genes can be amplified. Clones can be selected up to 10 nM methotrexate or higher and can be shown to produce bioactive mature G-TsF by immunoblotting and the thymocyte proliferation assay as described in the EXAMPLE.

4.3.2.2. Second variant: with p XMT3neo7/SUP40-1

In a further construction the full-length SUP40 cDNA (see Fig. 2c) was inserted into the COS-and CHO-cell expression vector pXMT3.neo7. This vector is a modification of pXM (Y. Yong et al., Cell 47 [1986] 3-10), which contains the same basic elements as p91023(B), although assembled differently. Therefore, as p91023(B), pXM alsoconferscells the elements necessary for replication of linked DNA in procaryotic and eucaryotic hosts and in addition the strong adenovirus major late promoter for expression of cDNAs cloned into a site close to the promoter and its linked elements. pXMT3.neo 7 was derived from pXM by insertion of multiple cloning site and the bacterial neo gene for convenient selection of transfected cells with the antibiotic G418.

The construct containing the full-length SUP40-cDNA in pXMT3.neo7 in the right transcriptional orientation, named pXMT3.neo7/SUP40-1 (see Fig. 3b) and transfected into E. coli HB 101, has been deposited on August 27, 1987 under Accession Number DSM 4226 with the Deutsche Sammlung von Mikroorganismen (DSM), Grisebachstr. 8, D-3400 Göttingen, German Federal Republic and converted on November 6, 1987 to a deposit under the Budapest Treaty on the International Recognition of the Deposit of Microorganisms for the Purposes of Patent Procedure (1977), with the same Accession Number.

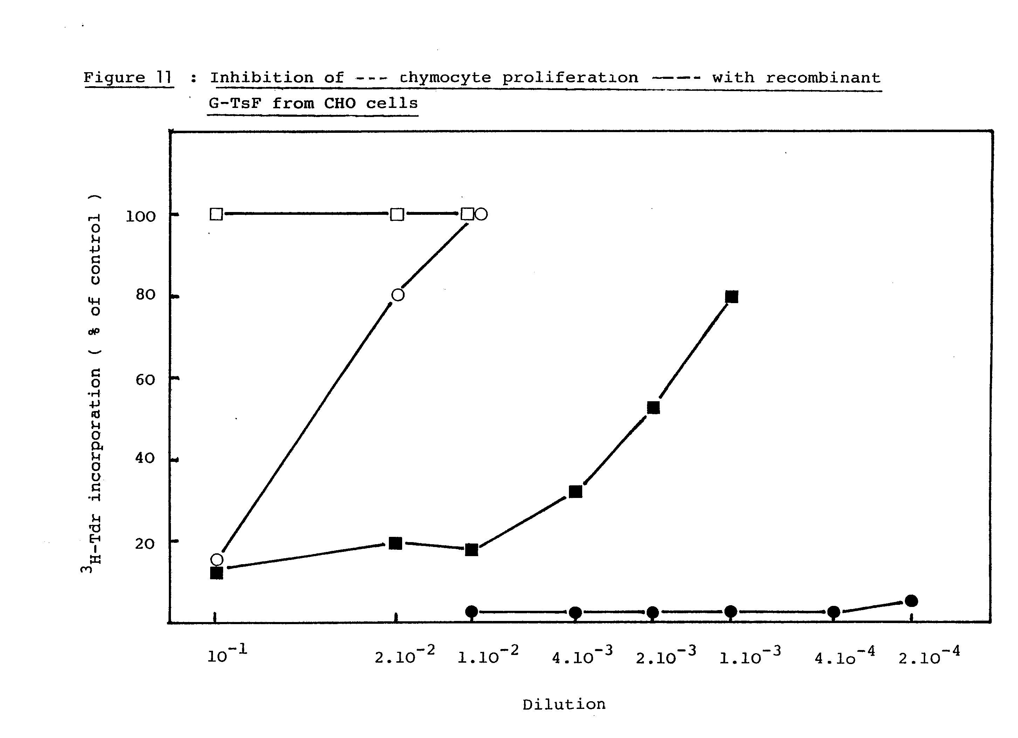

This plasmid has been transfected into the dihydrofolate-deficient CHO cell line DUKX and clones containing the plasmid DNA have been first selected by 0.5 mg/ml G418. Further selection for amplified dihydrofolate reductase sequences has been performed using α(-)medium and increesing concentrations of methotrexate as described above and in the EXAMPLE. It could be unambigously demonstrated that cell clones selected with 10 nM methotrexate produced recombinant mature human G-TsF by SDS-PAGE and immunoblotting of protein secreted by the cells (Figure 10). This material was also bioactive in the thymocyte proliferation assay with an estimated specific activity - - - - - - - (Fig. 11) which is close to the activity of material isolated from human glioblastoma cells. The cells selected in 10 nM methotrexate, dubbed CHO/-SUP40-1/10MTX - - - have been deposited on November 11 , 1987 under Accession Number ECACC 87111101 under the Budapest Treaty with the European Collection of Animal Cell Cultures, Porton Down, Salisbury, Wiltshire SP4 OJG, United Kingdom.

4.3.3. Expression in E. coli

The plasmid used for expression in E. coli is based on the lambda promoter. An oligonucleotide was synthesized and the 5′-end of the G-TsF sequence ligated with the NdeI site of the vector.

The resultant plasmid, named $pP_LS1$-SUP25-1 (see Figs. 7a and 7b), transfected into E. coli HB 101, has also been deposited on August 27, 1987, under Accession Number DSM 4225, with the Deutsche Sammlung von Mikroorganismen (DSM), and converted on November 6 , 1987 to a deposit under the Budapest Treaty,with the same Accession Number.

This construct was used to transfect E. coli strain W3110 lambda Y139 which produces a temperature-sensitive lambda receptor. The expressed product was extensively characterized. As can be seen from Fig.8 the recombinant G-TsF from E. coli has been purified to homogeneity, giving a single band in SDS-PAGE. In addition, protein sequencing revealed the correct amino-terminal amino acids in the material, thus demonstrating unambiguously that G-TsF may be efficiently expressed in procaryotic systems.

Fig. 9 indicates that although at a low level, some biological activity could be obtained with the recombinant material from E. coli. The low specific activity suggests that the bioactive dimeric form of G-TsF cannot form spontaneously from denatured monomeric G-TsF.

For expression in E. coli a methionine residue must be inserted in front of the mature G-TsF cDNA. Met-G-TsF is also part of the present invention. The insertion of a methionine residue may be effected in known manner.

4.3.4. Expression in other systems

Expression in further systems is of course possible using conventional techniques, which may result in improved efficacy and yields, e.g. starting from the above clones and using gene amplification techniques via selection with increasing concentrations of methotrexate.

It should be noted that the protein produced may be in an insoluble form which must be solubilized, or in some other form which may require refolding by conventional methods (see e.g. T.E. Creighton, Progr.Biophys.Molec.Biol. 33 [1978] 231-297).

G-TsF is secreted as a latent protein, possibly associated with portions of its precursor part, and with a binding protein, and may thus require some further processing, such as disruption of a noncovalent complex, for full biological activity. This processing may e.g. take the form of prior acidification of the supernatant from producing cells by e.g. dialysis against acetic acid or ammonium bicarbonate. Alternatively, alkali or chaotropic agents also come into consideration as activating agents. Preferred is acidification with e.g. acetic acid.

4.3.5. Although this aspect is not completely understood, the precursor peptide appears to be of major importance in successful expression of a biologically active product. It could be helpful or even essential in folding of the molecules, formation of disulfide bridges and proteolytic cleavage. After proteolytic release of mature G-TsF the precursor part peptide may still play an important role in e.g. potentiating the biological function of G-TsF, perhaps in relation with cell attachment, or the precursor part peptide may after proteolytic cleavage form the noncovalent complex alluded to above (see 4.3.4.) with the dimer of two subunits of mature G-TsF, perhaps together with a further component such as the protease involved in cleavage of the precursor. According to one line of thinking, unregulated epithelial cell growth may be a result of failure to activate the latent form of autocrine G-TsF.

Whatever the above uncertainties, since activation in vivo is a crucial regulatory step in the target specificity of G-TsF action, and the precursor clearly is of major importance in this activation process, the importance of the present invention may reside even more in the provision of the sequences for the precursor part peptide than in the sequences for the mature G-TsF.

The full precursor protein could also be synthesized as such in E. coli, be folded and dimerized, and the mature peptide later recovered by proteolytic cleavages. Equally well, non-cleaved natural or mutated precursor forms can be obtained from cells, e.g. CHO cells transfected with corresponding cDNAs and be cleaved after purification of the precursor. Hybrid forms of the precursor can be expressed by adding unrelated pre-pieces, for example those from interleukin-2 or GM-CSF, to certain internal positions of the precursor. These hybrid proteins may be more efficiently secreted and may or may not be correctly cleaved in the cells. Uncleaved forms may also be isolated from the cell lines and cleaved in vitro. The protease cleavage site could be mutagenized to yield sequences recognized by different proteases. Again, uncleaved precursors containing these modified cleavage sites could be isolated and cleaved by the respective enzymes.

The following Example illustrates the invention without being meant to be limitative:

5. EXAMPLE

5.1. PURIFICATION OF G-TsF

5.1.1. Glioblastoma cells SN 308 (A. Fontana et al., J.Immunol. 132 [1984] 1837-1844) are grown in tissue culture flasks or in multitrays (Nunc). The medium is Dulbecco's Modified Eagle' s Medium with 10% fetal calf serum and 300 microg/ml L-glutamine. After confluency is obtained the cells are washed with Hank's solution, treated with Trypsin/Versen (Gibco) and seeded at a density of 8 $\times 10^6$ cells/175 $cm^2$ flask or 4 $\times 10^8$ cells/multitray. After confluency has been achieved the cells are incubated for 3 days in serum-free medium with 1 microg/ml indomethacin. Then the supernatants are collected and used for purification of G-TsF as described in step 5.1.2. Alternatively, the cells are harvested 1 day after confluency and are used for mRNA isolation as described in step 5.2.1.

5.1.2. 1 liter of serum-free supernatant (altogether 1.1 $\times 10^5$ units in the thymocyte assay) is concentrated to 139 ml in the Pellicon® cassette system and a PTGC® membrane (Millipore), and diafiltrated against 5 volumes of 10 mM Tris.HCl, pH 7.5.

5.1.3. The sample is then chromatographed on a hydroxylapatite column:

dimensions: 1.6 $\times$ 24 cm; 49 ml
buffer A: 10 mM tris.HCl, pH 7.5 + 10 microM $CaCl_2$
buffer B: 0.5 M sodium phosphate, pH 7.5
flow rate: 2 ml/min

The biological activity elutes at 100-250 mM phosphate (buffer B)

5.1.4. This is followed by reversed-phase chromatography on Pro-RPC® (Pharmacia) with trifluoroacetic acid/acetonitrile as an eluent:

dimensions: HR 5/10; 2 ml
buffer A: 0.1 % trifluoroacetic acid in water
buffer B: 0.1 % trifluoroacetic acid in acetonitrile

0.1 % trifluoroacetic acid is added to the pool with the biological activity (29 ml), the solution is centrifuged and the supernatant (11.4 mg protein) is added to the column. The biological activity elutes at 1-25% buffer B (under conditions of excess protein).

5.1.5. Rechromatography on Pro-RPC™ the pool of biological activity is diluted 1:5 with buffer A and added to the column. The biological activity is eluting at 30-32 % eluent B.

5.1.6. Cation-exchange chromatography on Mono-S® (Pharmacia):

dimensions: HR 10/10, 8 ml
buffer A: 25 mM ammonium formiate, 5o % 2-propanol, pH 4.0
buffer B: 500 mM NaCl in buffer A

The pool with the biological activity is diluted 1:10 with buffer A. The activity is eluting at 68-69 % buffer B.

5.1.7. Reversed-phase chromatography on Pro-RP with trifluoroacetic acid/2-propanol:

dimensions: HR 5/2; 0.4 ml

buffer A: 0.1 % trifluoroacetic acid/water

buffer B: 0.1 % trifluoroacetic acid/2-propanol

The pool with the biological activity is diluted 1:5 with buffer A and added to the column. Elution of the biological activity takes place at 24-26 % of buffer B. Chromatography is started by - - - - - addition of the sample in buffer A followed by gradient elution with increasing concentrations of buffer B.

The result is shown in Fig. 4. The peak fraction from this column chromatography has the following characteristics:

- The proliferation of stimulated thymocytes is half-maximally inhibited at a dilution of 1:5000 in a 200 microl test sample. This is equivalent to an activity of about 25 000 units/ml.

  The test method is as follows: samples of 50 microl of supernatant at various dilutions are added to $6 \times 10^5$ thymocytes from C3H/HeJ mice - - - - - suspended in 150 microl of RPMI medium supplemented with 300 microg/ml L-glutamine, $1 \times 10^{-5}$ M 2-mercaptoethanol and 5 % fetal calf serum. The cells are incubated in flatbottomed microtiter plates for 72 hours in the presence of Concanavalin-A (0.5 microg/well). Sixteen hours before harvest 0.5 microCi of [$^3$H]-thymidine (5.0 Ci/mmol) is added per well. The results are expressed as percent suppression compared with the Concanavalin-A response of thymocytes treated with a medium control. For standardization purposes percent suppression can be plotted to give a standard curve from which conversion may be made to units of G-TsF activity. One unit of G-TsF is defined as the amount of G-TsF in 1 ml final assay medium which causes half-maximal inhibition in the assay.

- The interleukin-2 - dependent proliferation of antigen-specific T helper cells is also inhibited over 90 % at concentrations of G-TsF of $10^{-10}$M (see Fig. 5, part B).

  The test method is as follows: $2 \times 10^4$ T-helper cells OVA-7T are cultivated in microtiter plates in the presence of various concentrations of IL-2 (Amersham, ARN 1010. batch 10) and of test fractions. The medium is Iscove's Complete Medium (Behringwerke Cat.No. 7852), $5 \times 10^{-5}$ M 2-mercaptoethanol and 300 microg/ml L-glutamine. The incorporation of 3H-Tdr (1 microCi/well) is measured over 16 hours. The results are measured as cpm values of 3H-Tdr-uptake and expressed as percentage inhibition.

- Lyophilisation in the presence of carrier followed by SDS-PAGE (gel gradient 5-15 %) and silver staining gives a visible band of molecular weight 12.5 kd (extrapolated from standards with 93, 67, 45, 31, 21 and 14 kd)(see Fig. 4). The concentration of protein is estimated to be 0.5 microg/ml on the basis of the quantities of standard used, corresponding to a specific biological activity of the protein of $5 \times 10^7$ units/mg in the thymocyte proliferation assay and a half-maximal inhibition of the assay at a concentration of $10^{-11}$M (see Fig. 5, part A).

Taken together these data on the specific activity of the purified protein, together with the demonstration of a single brand in SDS-PAGE, unambiguously demonstrate that biologically active G-TsF has been purified to homogeneity.

## 5.2. CLONING AND SEQUENCING OF cDNA

### 5.2.1. Isolation of mRNA

The cells from 50 plates - - - grown as described in step 5.1.1. are scraped from the plates, centrifuged, and the pellet is suspended in 40 ml of a lysing solution (6M urea, 3 M LiCl, 50 mM sodium acetate pH 5.0, 200 microg/ml heparin, 0.2 % sodium dodecyl sulfate) and homogenized thrice for 30 seconds in a Sorvall Omnimix and stored at 4° C overnight. After centrifugation at 10 000 rpm and 4° C - - - - - - the precipitate is washed once with 8 M urea, - - - - - - - 4 M LiCl, then dissolved in 50 ml of acetate buffer (200 mM sodium acetate pH 5.0, 0.2 % sodium dodecyl sulfate, 1 mM EDTA) and extracted with water-saturated phenol. The aqueous phase is separated after centrifugation and extracted again with a 1:1 mixture of phenol and chloroform. The RNA is precipitated with 2 volumes of ethanol overnight at - 20° C. After centrifugation for 10 minutes at 10 000 rpm the precipitate is dissolved in 10 ml TES. The solution is brought to /.5 M NaCl and run over a column with 500 mg oligo(dT)-cellulose (PL, type 7). After an intensive wash with 0.5 M NaCl, 10 mM Tris.HCl pH 7.5, 1 mM EDTA, the bound poly(A)-containing mRNA is eluted with sterile water. 0.3 M sodium acetate pH 7.0 and 2.5 volumes of ethanol are added, the mRNA is precipitated overnight at - 20 °C and the solution centrifuged at 10 000 rpm. The precipitate is dissolved in water and brought to a concentration of about 1 microg/microl. Approximately 40-60 microg mRNA are recovered from 50 14-cm plates.

### 5.2.2. Synthesis of cDNA

The first cDNA strand is synthesized from 20 microg nRNA in 200 microl of a solution containing 50 mM Tris.HCl pH 8.3, 50 mM KCl, 8 mM $MgCl_2$, 1 mM dithiothreitol, 30 microg/ml oligo(dT), 0.1 microg/ml actinomycin D, 1 mM of each of dATP, dCTP, dGTP and dTTP, 1 unit/microl RNAse inhibitor RNAsin ® (Promega) and 1.5 unit/microl avian myeloblastosis virus reverse transcriptase (Life Sciences). Incubation is effected for 4 hours at 42° C. The reaction is stopped with 1/10 volume 0.5 M EDTA and run over a Pasteur pipette filled with Sephadex G 150 in order to remove the unincorporated triphosphates, actinomycin D and salts. 0.3 M sodium acetate pH 7.0 is added to the eluted cDNA/mRNA hybrid and the mixture precipitated with 2.5 volumes of ethanol overnight at - 20° C. The precipitate is recovered by centrifugation, washed once with 80 % ethanol and dissolved in 20 microl TEN.

The second strand is synthesized by the method of U. Gubler and B.J. Hoffman, Gene 25 (1983) 263-269 with the following modifications in a 400 microl reaction mixture consisting of 20 mM Tris.HCl pH 8.0, 5mM $MgCl_2$, 10mM $(NH_4)_2SO_4$, 100 mM KCl, 0.05 mM each of dATP, dCTP, dGTP and dTTP, 0.1 M dithiothreitol, 20 microCi $\alpha$[$^{32}$P]dCTP (Amersham, 3000 Ci/mmole), 20 microl RNAse H (Bethesda Research Laboratories, 3 units/microl), 10 microl DNA polymerase I (New England Biolabs, 10 units/microl) and the hybrid cDNA/mRNA obtained above. Incubation lasts for 90 minutes at 12°C and 3 hours at 22°C. The reaction is stopped with 1/20 volume 0.5 M EDTA and the low molecular weight components are separated over a Sephadex G150 column. The double-stranded cDNA is then precipitated with ethanol and dissolved in 20 microl TEN.

The cDNA is then methylated at the EcoRI sites by treatment for one hour at 37°C in 50 microl of 50 mM Tris.HCl pH 8.0, 0.1 mM EDTA, 80 microM adenosyl methionine (Sigma) and 5 microl EcoRI methylase (New England Biolabs, 20 units/microl).

Further, any hairpin loop formed is cut with nuclease S1 by treatment for 30 minutes at 22°C in 400 microl of 250 mM NaCl, 30 mM sodium acetate pH 4.5, 1 mM $ZnSO_4$ and 10 units S1 nuclease (PL). After extraction with phenol/chloroform 1:1 the cDNA is precipitated with ethanol, dissolved in 100 microl TEN and small DNA fragments are discarded by passage through a G150 column. After ethanol precipitation the cDNA is dissolved in a small volume (10-20 microl) TEN.

The ends of the cDNA are then prepared for linker ligation by incubating with T4 DNA polymerase for 30 minutes at 37°C in 40 microl of 33 mM Tris-acetate pH 7.9, 66 mM potassium acetate, 10 mM magnesium acetate, 2.5 mM dithiothreitol, 0.1 mM each of dATP, dCTP, dGTP and dTTP and 4 microl of T4 DNA polymerase I (New England Biolabs, 10 units/microl). The enzyme is inactivated by treatment for 10 minutes at 70 °C.

Synthetic EcoRI linkers (New England Biolabs) are then kinased at their 5'-ends as follows: 20 microg of linker DNA are incubated for 40 minutes at 37°C in 200 microl of 70 mM Tris.HCl pH 7.5, 10 mM $MgCl_2$, 5 mM dithithreitol, 2 mM ATP and 10 microl polynucleotide kinase.

The 5'-phosphorylated linkers are then ligated to the cDNA: the heat-inactivated cDNA reaction mixture is brought to 100 microl and the following concentrations: 35 mM Tris.HCl pH 7.5, 7 mM $MgCl_2$, 1 mM ATP and 5 mM dithiothreitol. 1.6 microg of kinased EcoRI linkers is added and ligation effected overnight at 15°C with 10 microl T4 DNA ligase (New England Biolabs). The ligase is then inactivated at 70°C for 10 minutes and the EcoRI linkers completely cleaved by treatment for 4 hours at 37°C with a large excess of EcoRI (New England Biolabs, 1000 units). The enzymes are extracted with 1 volume of phenol and the cDNA precipitated with ethanol. The monomeric linker fragments can be separated from the cDNA over a Sepharose 4B column. cDNA of more than 300 bp elutes in the exclusion volume, is precipitated with ethanol and dissolved in a small volume of TEN. The cDNA is then size-fractionated on a 1% Sea-plaque agarose gel in electrophoresis buffer (40 mM Tris.acetate pH 7.8, 1 mM EDTA) at 50 V, stained with ethidium bromide, and the DNA fragments larger than 1600 bp cut out from the gel. HinfI fragments of pBR 322 are used as size markers (J.G.Sutcliff, Nucl. Acids Res. 5 [1978] 2721-2728). An equal volume of TE and 3M sodium acetate pH 7.0 are added, the agarose is melted 5 minutes at 70°C and cooled for 5 minutes to 37°C. The agarose is extracted twice with an equal volume of phenol and the cDNA recovered from the aqueous phase by precipitation with ethanol.

5.2.3. Cloning into lambda gt10

The size-fractionated cDNA is cloned into phage lambda gt10 (T.V. Huynh et al., DNA Cloning, Glover Ed.,IRLPress, vol. 1 [1985] 49-78) Lambda gt10 arms dephosphorylated with phosphatase may be obtained from Stratagene Cloning Systems. 1 microg of lambda gt10 arms are ligated in a total volume of 8 microl, as described under 5.2.2., with the total cDNA obtained from 20 microg mRNA, using 1.6 microl T4 DNA ligase. The phages are then incubated with a packaging extract (Gigapack, Stratagene Cloning Systems) following the manufacturer's instructions and E.coli cells C600 hfl (Stratagene) are infected. The packaging of the phages is taking place with 20 microl freeze thaw and 30 microl sonic extract for 2 hours at 22°C. The phages are then diluted with 1 ml dilution buffer (5.8 g NaCl, 2 g $MgSO_4$. 7 $H_2O$, 50 mM Tris.HCl pH 7.5) and stabilized with 40 microl of chloroform. A fresh overnight culture of C600 hfl is then centrifuged and suspended in one-half volume 10 mM $MgSO_4$. 1 ml of this suspension of bacteria is infected with one-third of the phage suspension for 20 minutes at 37°C and the E.coli cells are plated in 30 ml Top-Agar in BBL®medium - - - - - - - - (0.8 % Bactoagar Difco, 10 g Trypticase, - - - - 5 g NaCl pH 7.2) on 1.5 % agar in a 245 × 245 mm biological testing dish (Nunc) and incubated for 5-6 hours at 37°C. The Top-Agar with the plaques is scraped off and shaken overnight in 20 ml phage buffer at 4°C. The agar is centrifuged off at 6000 rpm and the eluted phage suspension stabilised with 800 microl of chloroform. In this manner 500 000 recombinant phages are obtained from 3 plates, containing cDNA inserts over 1600 pb in length.

5.2.4. Synthesis of radioactive oligonucleotide probes

5.2.4.1. The oligonucleotides for Probe A and Probe B as shown in Fig. 1 are prepared using an Applied Biosystems DNA Synthesizer in accordance with the manufacturer's instructions (for Probe A the assumed amino acid at position 22, Ala, was found later to be wrong but this was of no consequence for the experiment).

5.2.4.2. They are then radioactively labelled as follows:

a) Probe A: 100 microg each of the two Probe A oligonucleotides are heated at 70°C for 10 minutes in 18 microl water, then 2 microl of a solution of 1 M NaCl, 100 mM Tris.HCl pH 7.5 are added and the probes are hybridized to each other for one hour at 37°C and 2 hours at room temperature. The mixture is then brought to 50 microl and the following concentrations are established: 50 mM Tris. HCl pH 8.0, 6 mM $MgCl_2$, 5 mM dithiothreitol, 0.05 mM each of dATP, dGTP and dTTP, and 100 microCi of $\alpha$[$^{32}$P]dCTP (Amersham, 3000 Ci/mMol). After addition of 2 microl of DNA polymerase I Klenow fragment the mixture is incubated for 45 minutes at room temperature and the unincorporated nucleotides are separated from the syntheized double-stranded DNA fragment over a Sephadex G25 column. 1 $\times$ $10^8$ to 2 $\times$ $10^8$ cpm/100 nanog of Probe A are obtained.

b) Probe B: this is radioactively labelled with 100 microCi $\alpha$[$^{32}$P]ATP (Amersham, 300 Ci/mMol) and 2 microl of polynucleotide kinase (Boehringer, 11 units/microl) in a 20 microl kinasing mixture, under conditions as described under 5.2.2. 0.5 $\times$ $10^7$ to 1 $\times$ $10^7$ units/100 nanog are obtained.

5.2.5. Screening and selection of G-TsF clones

About 100 000 recombinant phages (see 5.2.3.) are plated, after infection of E. coli C600 in 0.7 % Top-Agarose, in BBL® medium on 10 $\times$ 14 cm Petri dishes with 1.5 % agar. After incubation for 6 to 9 hours at 37°C the resultant plaques are replicated on Nylon filters (Pall) following the manufacturer's instructions. Two replicas are prepared. The DNA on the filters is first denatured with 0.5 N NaOH, 1.5M NaCl solution for 5 minutes and then neutralized with 3M Na-acetate pH 5.5 for 5 minutes. Finally, the filters are air-dried, dipped into chloroform for 30 seconds, air-dried and baked for 1 hour at 80° C.

The filters are then washed for 2 hours at 65° C in a solution of NET with 1% SDS and prehybridised for 4 hours at 42° C in a solution of NET with 0.2 % SDS, 0.1 % Ficoll, 0.1 % polyvinylpyrrolidone, 0.1 % calf serum albumin and 100 microg/ml of denatured salmon sperm DNA.

Hybridisation is effected in a solution of NET with 0.2 % SDS, 0.1 % Ficoll, o.1 % polyvinylpyrrolidone, 0.1 % calf serum albumin, 100 microg/ml yeast tRNA and o.05 % sodium pyrophosphate overnight at 42° C.

The first replica is hybridized with 2 $\times$ $10^6$ cpm/ml of Probe A, the second replica with o.5 $\times$ $10^6$ cpm/ml of Probe B. After hybridization has been effected the plates are washed twice for 30 minutes with a large volume of NET with 0.2 % SDS at room temperature. The temperature is then progressively increased to 42°C, 47°C, 52°C and 57°C and washing effected with the same solution twice for 15 minutes. After each washing step the filters are exposed for 16 to 48 hours on Kodak XAR-5 X-ray film with intensifier screens.

Clones hybridizing with both probes could be identified on the filters after washing at 52°C. An area corresponding to these clones is picked out of the plate and the phages are eluted in phage buffer. Since the area picked still contains a number of different recombinant phages, the eluted phages are used for renewed infection of E. coli C600 and the phages plated at low density. Replica are made again on nylon filters as described above, and the filters hybridized with the corresponding probes. A single plaque may now be picked from the plate. It is eluted in 1 ml of phage buffer and the resultant phage suspension is kept at 4° C.

After subcloning the cDNA insert of this clone (dubbed lambdaSUP25) into pBS.M13 (see below) a labelled RNA probe was obtained by transcribing the pSUP25 subclone with T3 RNA polymerase.

This is performed in a 10 microl assay containing 40 mM Tris.HCl pH 8.0, 8 mM $MgCl_2$, 2 mM spermidine, 50 mM NaCl, 75 mM dithiothreitol, 1 mM each of dATP, dGTP and dUTP, 25 units RNasin®, 50 micro Ci$\alpha^{32}$P dCTP (400-800 Ci/mmole), 1 microg linearized plasmid DNA and 10 units of T3 RNA polymerase. Incubation time is 30minutes at 37° C.

Approximately 1 $\times$ $10^6$ cpm of probe per ml of hybridization solution were used toscreen further replicas prepared after additional plating of the cDNA library as described above. Two further clones are isolated from this screen (lambdaSUP40 and lambdaSUP 42, see Fig. 2c).

5.2.6. Confirmation of identity of G-TsF clone

5.2.6.1. Recloning into sequencing vector pBS.M13

In order to confirm that the cDNA fragments obtained under 5.2.5. contain cDNA coding for full-length G-TsF it must be sequenced. First a small amount of phage DNA is isolated. 50 microl of the phage suspension obtained under 5.2.5. are used to infect a 100 microl suspension of E.coli C600 cells in 10 mM $MgSO_4$ for 15 minutes at 37° C. The resultant mixture is added to 20 ml of BBL® medium containing 0.2 % maltose and 10 mM $MgSO_4$ is agitated - - - - - for 4 to 7 hours at 37°C. 2 ml of chloroform are added for 30 minutes to the partially lysed culture, then it is centrifuged for 10 minutes at 5000 rpm and the supernatant is incubated for 60 minutes at 37° C with 30 microl of DNAse I (1 mg/ml) and 40 microl RNAse (5 mg/ml). The phages are precipitated at 4°C overnight by addition of an equivalent volume of 20 % polyethylene glycol 6000, 2 M NaCl, 2 g/l $MgSO_4$.7 $H_2O$, 50 mM Tris.HCl pH 7.5. The pellet obtained after centrifugation for 20 minutes at 5000 rpm is taken up in 500 microl phage buffer and extracted once with 500 microl of chloroform. The phage DNA in the aqueous phase is then treated with 3.8 microl of 20 % SDS and 7.5 microl of /.5 M EDTA at 68°C for 15 minutes, the proteins in the solution are extracted with phenol, and the DNA precipitated with ethanol.

The DNA is then dissolved in a small volume of TE. One half of the resultant phage DNA is cleaved with EcoRI in a 10 microl reaction mixture and the inserted cDNA fragment separated from the lambda arms over a Sea-Plaque agarose gel as described under 5.2.2. The band is cut out and the DNA recovered from the agarose as described under 5.2.2.by phenol extraction and ethanol precipitation. About 50 ng cDNA fragment are ligated with 50 ng pBS.M13 vector (Stratagene Cloning System) in a 10 microl ligation mixture as described under 5.2.2. For this the pBS.M13 vector must first be cleaved with EcoRI and dephosphorylated at the 5′ ends with alkaline phosphatase from calf intestine (Boehringer) according to the manufacturer's instructions. 1 to 2 microl of the ligation mixture are then transfected into a 100 microl suspension of competent E. coli JM 109.

Competent JM 109 cells may be obtained by standard methods such as methods described in T. Maniatis et al., Molecular Cloning: A Laboratory Manual, Cold Spring Harbor Laboratory, New York (1982). 100 microl of cell suspension are thawed over ice and 2 microl of ligation mixture are added. Incubation takes place for 20 minutes over ice, the cells are then incubated for 45 seconds at 37° C and agitated for 1 hour at 37° C in 1 ml SOC medium (2% Bacto-Tryptone, 0.5 % yeast extract, 10 mM NaCl, 2.5 mM KCl, 10 mM $MgCl_2$, 10 mM $MgSO_4$, 20 mM glucose). The bacteria are pelleted by - - - - centrifugation, suspended in 100 microl of SOB medium (SOC medium without the glucose) and spread over a 1.5 % agar plate in SOB medium with 50 microg/ml ampicillin. When the plate has been pretreated with 100 microl of a solution containing 4 mg 5-bromo-4-chloro-3-indolyl-β-D-galactopyranoside and 50 mg isopropyl-β-D-thiogalactopyranoside per ml of dimethylformamide, then the β-galactosidase contained in the vector causes a blue color of the E. coli colonies. Colonies with insertions are colorless. These colonies are seeded into 6 ml cultures and grown overnight. The growth medium may be SOC medium with 50 microg/ml ampicillin. Plasmid DNA is isolated with the "boiling method" as described in Maniatis (1982, supra).

5.2.6.2. Sequencing of cDNA fragments

One third of the plasmid DNA obtained from 6 ml of culture medium is denatured by treatment for 5 minutes with 1/10 volume 2 N NaOH, 2 mM EDTA; 1/5 volume 5 M ammonium acetate is added and the DNA precipitated with 2 volumes ethanol. The precipitate is dissolved in 8 microl water, 1.5 microl of reaction buffer (0.5 M NaCl, 0.1 M Tris.HCl pH 7.5, 0.1 M $MgCl_2$, 1 mM EDTA) is added, and appropriate oligonucleotide primers are hybridized with the cDNA. After addition of the various mixtures of deoxynucleotide- and dideoxynucleotide-triphosphates the DNA is sequenced according to the method of F. Sanger et al., PNAS 74 (1977) 5463-5477 using Klenow DNA polymerase 1.

The sequencing strategy to confirm the identity of the SUP25 cDNA is illustrated in Fig. 2b. As a first primer Probe B (see 5.2.4.2.) can be used. It is possible to read a region of about 200 nucleotides thereby. This gives enough sequence information to synthesize two further sequencing primers:

- Primer 1: 5′-CCG TAT TTA TGG AGT TCA G-3′
- Primer 2: 5′-GGA GAA GCA GAT GCT TCT GGA T-3′

With these two primers it is possible to read the complete region covering the 112 amino acids of the mature protein (see Fig. 2a). The correct position of the mature peptide within the precursor protein is deduced from the determined amino-terminal peptide sequence and can also be inferred by comparison with the published sequence for TGF-β1 (R. Derynck et al., Nature 316 [1985] 701-704).

The full sequence of the cDNA between positions 1 and 1695 was derived after subcloning and sequencing of a series of overlapping cDNA fragments obtained by splitting the SUP25-cDNA with various restriction enzymes or by treating subclones with mung-bean nuclease and exonuclease III according to the method of S. Henikoff, Gene 28 (1984) 351-359).

In addition the SUP40 and SUP42 cDNAs were sequenced for 300 nucleotides from both ends and in the regions coding for the mature peptide using the internal sequencing primers described above. The sequenced regions were found to be identical to the SUP25 cDNA (see Fig. 2c and 2d).

The full sequence for the mature protein has 71 % similarity with TGF-β1, and the protein sequence is, as for TGF-β1, at the carboxy-terminal end of a precursor protein (see Fig. 2b). This follows from the presence of a termination codon at the 3′-end and of an arginine residue as part of a potential protease cutting site in front of the first amino acid in the mature protein.

5.3. Expression of recombinant G-TsF

5.3.1 Expression in CHO cells

5.3.1.1. Construction of expression vectors

The lambda SUP25 and lambdaSUP40 cDNAs (see Fig. 2c) are recloned into the COS-and CHO-cell expression vectors p91023B and pXMT3.neo 7.

p91023B is described in G.G. Wong et al., Science 228 (1985) 810-815.

pXMT3.neo 7 was devided from the pXM vector, which contains the same main elements as p91023B and is described in Y. Yang et al., Cell 47 (1986) 3-10.

In short, these vectors both contain elements permitting growth in E. coli and eucaryotic cells. The inserted cDNA may further be expressed in CHO and COS cells. They contain initiation sites for replication in procaryotes and eucaryotes, the enhancer element from SV40, the major late promoter of adenovirus followed by parts of the three leader sequences of the late transcript of adenovirus, an intron from an immunoglobulin gene, a polyadenylation site from SV40 and the VA genes of adenovirus which function together with the 5′-untranslated leader sequences in the control of translation.

In addition the vectors contain a mouse dihydrofolate reductase cDNA inserted between the cloning site, which is located after the immunoglobulin intron, and the polyadenylation sequence from SV40 (see Fig. 3a). Therefore, these vectors can also be transfected into CHO cells and the transferred genes amplified together with the dihydrofolate reductase cDNA by selection of subclones resistant to increasing concentrations of methotrexate (according to J. Haynes et al., Nucl.Acid.Res. 11 (1983) 687-706).

The pXMT3.neo 7 construct is derived from the pXM vector to facilitate the cloning procedure by inserting multiple cloning sites. Further, the bacteriel neo-gene under the control of the thymidine kinase promoter of Herpes sinplex virus is inserted between the SV40 origin of replication and the VA genes. This allows a first selection step after transfection of CHO cells using G418 sulphate (Gibco) according to F. Colbère-Garapin et al., J.Mol.Biol. 150 [1981], 1-14. In detail. the PstI-EcoRI region of the polylinker from pUC18 (C. Yanich-Perron et al., Gene 33 [1985] 103) was inserted into the pXM plasmid after cleavage with PstI and EcoRI, which removed a 36 bp fragment from pXM. This gave clone pXMT3. For the insertion of the neo-gene the 1882 bp fragment obtained from a partial PvuII cleavage of pAG60 (F. Colbère-Garapin et al., J.Mol.Biol. 150 [1981] 1-14) containing the thymidine kin ase promoter coupled to the neo-gene was inserted into StuI-cleaved pXMT3 and a plasmid containing the neo-gene and the adenovirus major late promoter in the same transcriptional orientation, pXMT3.neo 7 was selected (see Fig. 3b).

The recloning is effected as described under 5.2.6.1. into the EcoRI site of the vectors (see Figs. 3a and 3b).

5.3.1.2. Transfection of CHO cells and amplification of the G-TsF cDNA

Transfection of the dihydrofolate reductase - deficient CHO cell line DUKX-B (G. Urlaub and L.H. Chasin, PNAS 77 [1980] 4216-4220) was performed by electroporation according to E. Neumann et al., EMBO J. 1, [1982] 841-845. Cells were grown to near-confluency in α + medium (Yibco), washed once with PBS without Ca++ and Mg++ and released from the plates by incubation in PBS containing 0.5 mM EDTA and 0.5 mM EGTA for approximately 5 minutes. The cells were centrifuged and resuspended in TBS (25 mM Tris.HCl pH 7.5, 137 mM NaCl, 5 mM KCl, 0.7 mM $CaCl_2$, 0.5 mM $MgCl_2$ and 0.6 mM $Na_2HPO_4$). Following an additional centrifugation the cells were suspended in cold TBS and adjusted to $10^7$ cells/ml. Electroporation was performed with 100 microl aliquots of this suspension using an ISCO model 494 power supply and a Haefliger electroporation unit. Cells were incubated together with 5μg of linearized plasmid DNA for 30 minutes before the pulsing and for another 30 minutes thereafter. 5 pulses were delivered at five seconds intervalls. The power supply settings used were: 2000 V, 5W, 3mA, voltage limit between 600 and 1800 Volts as specified giving field strengths of 3000 to 9000 V/cm. The capacitors were selected to charge to 169 microF. Following the electroporation the cells were seeded into 75 cm² culture flasks in α+ medium. G418 was added 24 hours later at a concentration of 0.5 mg/ml and cell clones having incorporated the plasmid DNA containing the neo-gene were selected. The mixture of growing cell clones was released from the plates by incubation in PBS containing 0.5 mM EDTA and 0.5 mM EGTA and reseeded in α(-)-medium (Gibco) to select for the presence of dehydrofolate reductase in the cells. Further amplifications of the transfected genes was then achieved by selection after reseeding the cultures in increasing concentrations of methotrexate, --- starting with 1 nM and increasing to 3 and 10 nM.

5.3.1.3 Confirmation of production of bioactive recombinant human G-TsF by CHO cells

The cell clones selected in 1 nM and 10 nM methotrenate as well as a control culture transfected with the vectors without cDNA inserts and selected the same way were seeded at a cell density of 2 to 4 × $10^6$ cells / 75 cm² flask and grown to 90 % confluency. Then the cells were washed three times with PBS to remove the methotrexate and incubated for 48 hours with 6 ml of serum-free α-medium or α-medium containing 10 % fetal calf serum. After precipitation of the proteins from the serum-free culture supernatants with trichloroacetic acid, SDS-PAGE and immunoblotting was performed according to standard protocols as described by H. Towbin et al., PNAS 76 [1979] 4350-4354. The rabbit antiserum employed was obtained by immunizing rabbits with a synthetic peptide corresponding to the aminoterminal part of G-TsF. The peptide Ala-Leu-Asp-Ala-Ala-Tyr-Cys-Phe-Arg-Asn-Val-Gln-Asp-Asn-Cys-Cys-Leu-Arg-Pro-Leu-Tyr-Ile-Asp-Phe-Lys-Arg-Asp-Leu-Gly

(position marks: 5, 10, 15, 20, 25)

was synthesized on a Biosystem Peptide synthesizer according to the manufacturer's protocol and injected into rabbits according to standard procedures.

The immunoblotting experiments unambiguously identify the production of human recombinant mature G-TsF in the cell clones selected in 10 nM methotrexate. No G-TsF material was detectable under these conditions in the control cells and the cells selected in 1nM methotrexate (see Fig. 10).

The supernatants obtained in 10 % serum medium devoid of methotrexate were tested in the thymocyte proliferation assay as described in step 5.1.7. Whereas the supernatants from control cells and cells selected in 1 nM methotrexate revealed very little inhibitive activity, the medium obtained from the cell clones selected in 10 nM methotrexate showed ------ inhibition of thymocyte proliferation:-----------. Following acid activation of the G-TsF complex produced by the cell clones the clones selected with 10 nM ------------------- methotrexate caused half-maximal inhibition in the assay at a dilution-- beyond 1: 5000 ----------------- (see Fig. 11). This experiments definitely proves the production of bioactive recombinant human G-TsF by CHO cells transfected with the G-TsF cDNA. The specific activity calculated from the amount of G-TsF estimated to be present in the immunoblot (see Fig. 10) (approximately 100 ng/ml) is at least as high as the 5 × $10^7$ units/mg calculated for the material isolated from glioblastoma cells (see 5.1.3.).

Further, the inhibitory activity of the supernatant obtained from CHO cells transfected with pXMT3.neo7/SUP40-1 and selected at 10 nM methotrexate was completely neutralized in the thymocyte assay after incubation of methotrexate-free supernatant diluted 1:10 with 24 microg/ml of polyclonal anti-TGF-β1 antibodies (R & D Systems), whereas This ------ normally results in 87 % inhibition in the absence of antibodies. These antibodies have been shown to be cross-reactive for TGF-β1 and G-TsF; they neutralize natural G-TsF from glioblastoma cells.

5.3.2. Expression in E. coli

A part of the lambda SUP25 cDNA containing the coding region for the mature G-TsF peptide and 3'-untranslated sequences down to the Hind III site (see Fig. 2c) is cloned into $pP_LS1$. This vector allows expression of cDNA under the control of the lambda promoter. For the construction an oligonucleotide is first synthesized which connects the HaeIII site at position 1099-1102 of the G-TsF cDNA with the NdeI site in the vector in such a way that an ATG translation initiation codon is created. This is followed directly by the sequence coding for mature G-TsF of 112 amino acids in length (Figs. 7a and 7b). This construct ($pP_LS1$-SUP25-1) is transfected into E. coli strain W3110 lambdaY139 which produces a temperature-sensitive lambda repressor. The bacteria are then grown at 30°C. After the density of 0.6 $OD_{600}$ is attained the $P_L$ promoter is induced by raising the temperature to 42°C (this inactivates the lambda repressor). After 54 hours of incubation total E. coli protein is isolated and the recombinant G-TsF checked after separation over a polyacrylamide gel, using the polyclonal rabbit antibody to G-TsF described in 5.3.1.3. Thus the production of G-TsF could be demonstrated starting one hour after inactivation of the lambda repressor at 42°C (see Fig. 8).

The identity of the G-TsF expressed in E. coli was further checked by growing 6 l of culture medium containing E. coli with the recombinant plasmid to a density of $OD_{600} = 0.6$, inducing for 5 hours at 42°C and isolating the G-TsF as follows:

1) Extraction: The 8 g of cells obtained were suspended in 28 ml of buffer (10 mM tris.HCl pH 7.5; mM EDTA; 1.5 % SDS; and 0.5 % 2-mercaptoethanol), heated at 50°C for 20 minutes and then centrifuged for 15 minutes at 48 000 g. The supernatant was discarded and the pellet extracted again with the same buffer but containing 5 % SDS. Both supernatants were mixed and the viscosity reduced by energetic agitation.

2) Chromatography over Biogel: The extracted protein was fractionated over a Biogel column (Biogel P100,160-200 mesh, K 100/100) and eluted with buffer (composition as under 1) (120 ml, flow-rate 260 ml/hour). Aliquots of the fractions were separated by PAGE and immunoblotted.---------- Fractions 23 and 24 were merged, lyophilized, dissolved in 40 ml of distilled water and dialyzed for 6 days against distilled water. The dialyzed material (85 ml) was then brought to pH 2.15 with trifluoroacetic acid.

3) Reversed-phase f.p.l.c. over Pro-RPC (Pharmacia HR 16/10): The dialyzed fractions were centrifuged at 48 000 g for 10 minutes and the clear supernatant (9 mg protein) transferred to the column:

Buffer A: 0.1 % trifluoroacetic acid in water
buffer B: 0.1 % trifluoroacetic acid in acetonitrile
fraction sizes: 2 ml; flow-rate: 2 ml/min
gradient: 5-60 % in 45 minutes

The fractions containing G-TsF (fractions 43 and 44) were checked by immunoblotting, merged, given 5 mg mannitol as carrier and lyophilized.

4) Cation-exchange f.p.l.c. over Mono S (Pharmacia HR 10/10): The lyophilized product was dissolved in 2 ml of buffer A (25 mM ammonium formiate pH 4.0; 50 % 2-propanol) and transferred to the column:

Buffer B: 500 mM NaCl in buffer A
fraction size: 1 ml; flow-rate: 1 ml/min
gradient: 0-100 % in 60 minutes.

The fractions containing G-TsF were determined in SDS-PAGE after silver staining (Fig. 9). Fractions 31 and 32 (eluted with 175 mM NaCl, approximately 8 microg protein) were lyophilized with 1 mg mannite.

5) Reversed-phase f.p.l.c. over Pro-RPC (Pharmacia HR 5/2): The lyophilized product was dissolved in 1 ml of buffer A (0.1 % trifluoroacetic acid in water) and transferred to the column:

Buffer B: 0.1 % trifluoroacetic acid in acetonitrile fraction size: 1 ml; flow-rate: 1 ml/min gradient: 5-60 % buffer B in 45 minutes

The fractions are characterized by SDS-PAGE and silver staining of aliquots. The material from the fraction eluting at 36 % buffer B (approximately 1 microg protein) is used for amino-terminal sequencing.

6) Amino-terminal sequencing: 800 microl from fraction 26 of step 5) were transferred directly onto the glass filter of an Applied Biosystems gas-phase-sequenator (Run 155, 87 05 15). Degradation and amino acid identification were effected following the manufacturer's instructions. The result (Ala-Leu-blank-Ala-Ala-Tyr-blank-Phe-blank-Asn) confirms the identity and the correct amino-terminal sequence of the recombinant G-TsF expressed in E.coli).

7) An aliquot of fractions 31 and 32 of step 4) was tested in the thymocytes proliferation assay (see 5.1.7.) and shown to have the inhibitory activty (see Fig. 10) although half-maximal inhibition was observed only at concentrations of approximately $10^{-7}$M. This indicates that only a small fraction of the monomer G-TsF is able to spontaneously form the bioactive dimeric form.

6.

The present invention is thus making possible the preparation of large quantities of recombinant G-TsF for further study and therapeutic applications.

It is clear that G-TsF is a multifunctional peptide which, apart from its immunosuppressant effect, also controls the proliferation, differentiation and other functions in many types of cells.

G-TsF is useful in the indications mentionned under "1. BACKGROUND" for TGF-β2, CIF-B and G-TsF, primarily as an immunosuppressive, wound healing, bone forming and antiinflammatory agent, e.g. in tumor, transplant, autoimmune, osteoporosis, tissue injury and inflammation conditions.

The factor is biologically active mainly as the dimer of about 25 kd.

The immunosuppressant activity may for example be determined in vitro by the inhibition of lectin-stimulated proliferation of thymocytes and by the inhibition of the interleukin-2-dependent proliferation of murine antigen-specific T helper cells. In vivo assay may e.g. be effected in the alloreactive cytolytic T-cell model and in the encephalomyelitis model as described in S.H. Hefeneider et al., J. Immunol. 130 (1983) 222-227 and in A. Ben-Nun et al., J. Immunol. 129 (1982) 918-919. Further test models are described in Sandoz EP 159 289 G-TsF inhibits early activating mechanisms in T-cells, including IL-2-dependent steps. This activity is useful in controlling the human immune system in many indications ranging from organ transplantation to auto-immune diseases such as chronic polyarthritis and immune-dependent encephalitis.

Thus, concentrations of $10^{-11}$ M (based on the monomer) result in half-maximal inhibition of Concanavalin-A-induced thymocytes proliferation and interleukin-2-induced T-helper cell proliferation (see EXAMPLE)

Further, G-TsF increases connective tissue formation, and leads to fibrosis and angiogenesis. — — — — — It is thus useful in the treatment of skin lesions caused by trauma, burns, operations or senility. The capability of G-TsF to promote the formation of collagen makes it useful also for the treatment of, e.g., osteoporosis, where insufficient formation of collagen and other components of the bone matrix leads to pathogenesis.

The above tissue-forming activities may be shown in known test models, e.g. in the rat wound healing assay (M.B. Sporn et al., Science 219 [1983] 1329-1330) and in the rat muscle cell induction assay (S.M. Seyedin et al., PNAS 82 [1985] 2267-2271).

Further applications of interest include the use of G-TsF in the treatment of skin-graft donor sites, injuries to the cornea, bedsores and diabetic ulcers. Even a potential use in cancer therapy can be envisaged.

Further, knowledge of the full sequence for the mature and precursor G-TsF enables the preparation of recombinant G-TsF derivatives having modified, e.g. improved properties over those of the mature product.

It is quite possible that the precursor part as such has beneficial pharmacological activities of its own.

For the above indications the appropriate dosage will, of course, vary depending upon, for example, the G-TsF employed, the host, the mode of administration and the nature and severity of the condition being treated. However, in general, satisfactory results in animals are indicated to be obtained at daily dosages from about 0.3microgram per kg to about 15 micrograms per kg animal body weight. In larger mammals, for example humans, an indicated daily dosage is in the range from about 20 micrograms to about 1000 micrograms, e.g. 50 micrograms to 500 micrograms of G-TsF in mature form, conveniently administered, for example, in divided doses up to four times a day.

G-TsF may be administered by any conventional route for peptides, in particular topically, e.g. in the form of oint ments or suspensions, or systemically, e.g. in the form of parenteral injections or infusions.

The present invention also provides pharmaceutical compositions comprising G-TsF in association with at least one pharmaceutical carrier or diluent. Such compositions may be manufactured in conventional manner. Unit dosage forms contain, for example, from about 5 microg to about 500 microg of G-TsF.

Particularly preferred indications for G-TsF are wound healing, either surface of internal wounds, osteoporosis, organ transplantation and autoimmune diseases.

G-TsF may be combined with activating agents such as TGF-α, TGF-β1, EGF (epidermal growth factor) or other growth and differentiation factors. The amount of activating agent present depends directly upon the amount of G-TsF present in the activated compositions as administered to the recipient.

7. ABBREVIATIONS

CHO Chinese Hamster Ovary
CIF-B Cartilage-Inducing Factor B
DMEM Dulbecco's Modified Eagle's Medium
EDTA ethylene diamine tetracetic acid
EGTA ethyleneglycol-bis-(β-aminoethylether)-N,N,N',N'-tetraacetic acid
FPLC® fast protein liquid chromatography (Pharmacia)
G-TsF Glioblastoma-derived T-cell suppressor Factor
$^3$H-Tdr tritriated thymidine
IL-2 Interleukin-2
MIS Mullerian Inhibiting Substance
NET 750 mM NaCl, 5 mM EDTA, 50 mM Tris.HCl pH 7.5
PAGE polyacrylamide gel electrophoresis
PBS phosphate-buffered saline: 8g/l NaCl, 0.2g/l KCl, 1.15 g/l $Na_2HPO_4$, 0.2 g/l $KH_2PO_4$
SDS sodium dodecyl sulfate
TBS 10 mM Tris.HCl pH 7.5, 140 mM NaCl
TE 10 mM Tris.HCl pH 7.5, 1 mM EDTA
TEN 100 mM NaCl, 10 mM Tris.HCl pH 7.5, 1 mM EDTA
TES 10 mM Tris.HCl pH 7.5, 1 mM EDTA, 0.2 % SDS
TGF-β Transforming Growth Factor β (= TGF-β1)
TGF-β1 Transforming Growth Factor β1
TGF-β2 Transforming Growth Factor β2

**Claims**

1. A protein in substantially pure form having human G-TsF-like activity.
2. Substantially pure human G-TsF.
3. A protein having G-TsF-like activity whenever prepared by recombinant DNA techniques.
4. Recombinant G-TsF.
5. Recombinant mammalian G-TsF.
6. Human recombinant G-TsF.
7. Recombinant G-TsF precursor.
8. Recombinant G-TsF precursor part.

9. Recombinant mature G-TsF.

10. The protein having the full amino acid sequence indicated in Fig. 2d.

11. The protein having the amino acid sequence indicated in Fig. 2d before the arrow.

12. The protein having the amino acid sequence indicated in Fig. 2d after the arrow.

13. An allelic or a variant form of a protein of any one of claims 1 to 12.

14. An allelic variant, or a variant resulting from point mutation or larger modifications to enhance activity or production without changing the main functional and structural properties, of a protein according to any one of claims 1 to 12.

15. A deletion, insertion or substitution mutant of a protein of any one of claims 1 to 12.

16. A protein of any one of claims 1 to 15 in glycosylated form

17. A protein of any one of claims 1 to 15 in non-glycosylated form.

18. A protein of any one of claims 1 to 17 in dimeric form.

19. A protein as defined in claim 10 or 12 having in amino acid position 16 from the beginning of the mature sequence Ile instead of Cys and/or in position 25 Val instead of Lys.

20. Recombinant mature G-TsF whenever prepared using the G-TsF precursor part.

21. Mature G-TsF protein preceeded by a methionine residue.

22. A protein of any one of claims 1 to 21 having a molecular weight for the mature monomeric unit of 12 320 daltons disregarding any glycosylation.

23. A protein of any one of claims 1 to 21 having a molecular weight for the monomeric precursor of 45 540 disregarding any glycosylation.

24. A protein of any one of claims 1 to 23 having a specific activity in the thymocyte proliferation assay of at least $5 \times 10^7$ units/mg.

25. A mature protein of any one of claims 1 to 24 having more than 71 % amino acid sequence similarity with the amino acid sequence indicated in Fig. 2d after the arrow.

26. A precursor protein of any one of claims 1 to 24 having more than 30 % amino acid sequence similarity with the full amino acid sequence indicated in Fig. 2d.

27. A protein of any one of claims 1 to 26 for use in therapy.

28. A process for the preparation by recombinant DNA techniques of a protein having G-TsF-like activity, comprising the recovery of expressed protein from a host cell transformed with a gene coding for such protein.

29. A process according to claim 28 for the preparation of a protein having G-TsF-like activity comprising

a) construction of a vector which includes nucleic acid encoding such protein,

b) transformation of an heterologous host cell with the vector,

c) culture of the transformed host cell and

d) recovery of expressed protein from the culture.

30. A process for the preparation of bioactive dimeric G-TsF via the prior synthesis of the G-TsF precursor or of parts of the G-TsF precursor essential in folding and proteolytic processing.

31. A process for the preparation of a protein of claim 1 or 2 comprising a cation-exchange chromatography step, preferably over Mono-S®

32. A process according to claim 31 comprising chromatography on hydroxylapatite and Pro-RPC®, cation-exchange chromatography on Mono-S® and reversed-phase chromatography over Pro-RPC®.

33. A process according to claim 32 comprising

a) concentration and diafiltration of supernatants from cells from a natural source of G-TsF such as glioblastoma cells,

b) chromatography on hydroxylapatite,

c) reversed-phase chromatography over using trifluoroacetic acid/acetonitrile (2 × ) as an eluant,

d) cation-exchange chromatogrophy, preferably over Mono-S®, and

e) reversed-phase chromatography over Pro-RPC® using trifluoroacetic acid/ 2-propanol as an eluant.

34. A cDNA coding for a protein of any one of claims 1 to 27.

35. A cDNA of claim 34 having the nucleotide sequence indicated in Fig. 2d.

36. A cDNA of claim 35 having the nucleotide sequence indicated in Fig. 2d before the arrow.

37. A cDNA of claim 35 having the nucleotide sequence indicated in Fig. 2d after the arrow.

38. A cDNA capable of hybridizing with a nucleic acid of any one of claims 34 to 37 and coding for a protein having G-TsF-like activity, or a cDNA capable of hybridizing with the complementary strand of such nucleic acid.

39. A vector for expression of a recombinant protein of any one of claims 3 to 27 including nucleic acid encoding such protein.

40. A vector of claim 39 for eucaryotic expression.

41. A vector of claim 39 for expression in CHO cells.

42. The vector of claim 39 which is p91023(B)-SUP25-1.

43. The vector of claim 39 which is pXMT3.neo7/SUP40-1.

44. A vector of claim 39 for procaryotic expression.

45. A vector of claim 39 for expression in E. coli.

46. The vector of claim 39 which is $pP_L$SI-SUP25-1.

47. A cell line transformed with nucleic acid coding for a protein of any one of claims 3 to 27.

48. A cell line of claim 47 which is α CHO cell line.

49. The cell line of claim 47 which is CHO/ SUP40-1/10MTX.

50. A pharmaceutical composition comprising a protein of any one of claims 1 to 27 together with a pharmaceutically acceptable carrier or diluent.

51. A method of treatment comprising administration of a therapeutically effective amount of a protein of any one of claims 1 to 27 to a subject in need of such treatment.

52. Use of a protein of any one of claims 1 to 27 in therapy.

53. Use according to claim 52 in immunosuppression, wound healing, bone formation, or inflammation.

54. Use according to claim 52 in organ transplant or auto-immune conditions.

55. Use according to claim 52 in osteoporosis or tissue injury conditions.

56. Use according to claim 52 in the treatment of skin lesions caused by trauma, burns, operations or senility.

57. Use according to claim 52 in cancer therapy.

58. Use of a protein of any one of claims 1 to 26, or use of a cDNA ---- of ---- any one of claims 34 to 38, or use of a vector of any one of claims 39 to 46, or use of a cell of any one of claims 47 to 49, in the preparation of G-TsF derivatives.

## Figure 1: Oligonucleotides used for screening the cDNA library

**Probe A:**

```
    1                                   10                                      20
   Ala Leu Asp Ala Ala Tyr Cys Phe Arg Asn Val Gln Asp Asn Cys Cys Leu Arg Pro Leu Tyr Ala Asp

   **  **  *** **  *** **  *** **  *   *** *** *** **  **  *** *** **  *   **  **  **  *   **
5'-GCC CTG GAT GCT GCC TAC TGC TTC CGC AAT GTG CAG GAC-3'
                                    3'-TTA CAC GTC CTG TTG ACG ACG GAC TCC GGC GAC ATA CGA CTG-5'
```

**Probe B:**

```
    4                                   13
   Ala Ala Tyr Cys Phe Arg Asn Val Gln Asp

5'-GCI GCI TAC TGC TTC IGI AAC GTI CAI GA-3'
             T   T   T       T
```

Figure 2a: Complete nucleotide and amino acid sequences of mature human G-TsF

Protease cleavage site

↓

27 54

CGT | GCT TTG GAT GCG GCC TAT TGC TTT AGA AAT GTG CAG GAT AAT TGC TGC
Arg | Ala Leu Asp Ala Ala Tyr Cys Phe Arg Asn Val Gln Asp Asn Cys Cys

1 10

81 108

CTA CGT CCA CTT TAC ATT GAT TTC AAG AGG GAT CTA GGG TGG AAA TGG ATA CAC
Leu Arg Pro Leu Tyr Ile Asp Phe Lys Arg Asp Leu Gly Trp Lys Trp Ile His

20 30

135 162

GAA CCC AAA GGG TAC AAT GCC AAC TTC TGT GCT GGA GCA TGC CCG TAT TTA TGG
Glu Pro Lys Gly Tyr Asn Ala Asn Phe Cys Ala Gly Ala Cys Pro Tyr Leu Trp

40 50

189 216

AGT TCA GAC ACT CAG CAC AGC AGG GTC CTG AGC TTA TAT AAT ACC ATA AAT CCA
Ser Ser Asp Thr Gln His Ser Arg Val Leu Ser Leu Tyr Asn Thr Ile Asn Pro

60 70

243 270

GAA GCA TCT GCT TCT CCT TGC TGC GTG TCC CAA GAT TTA GAA CCT CTA ACC ATT
Glu Ala Ser Ala Ser Pro Cys Cys Val Ser Gln Asp Leu Glu Pro Leu Thr Ile

80

297 324

CTC TAC TAC ATT GGC AAA ACA CCC AAG ATT GAA CAG CTT TCT AAT ATG ATT GTA
Leu Tyr Tyr Ile Gly Lys Thr Pro Lys Ile Glu Gln Leu Ser Asn MET Ile Val

90 100

351

AAG TCT TGC AAA TGC AGC TAA
Lys Ser Cys Lys Cys Ser .

110

Figure 2b: Sequencing strategy

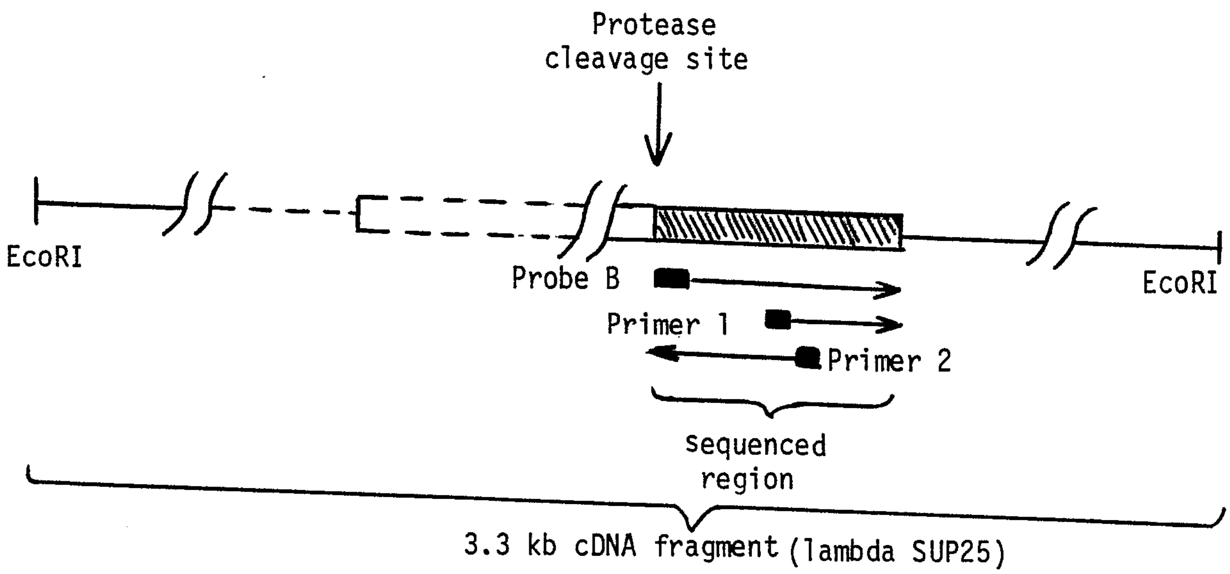

Figure 2c: Schematic diagram and partial restriction endonuclease map of G-TsF cDNA

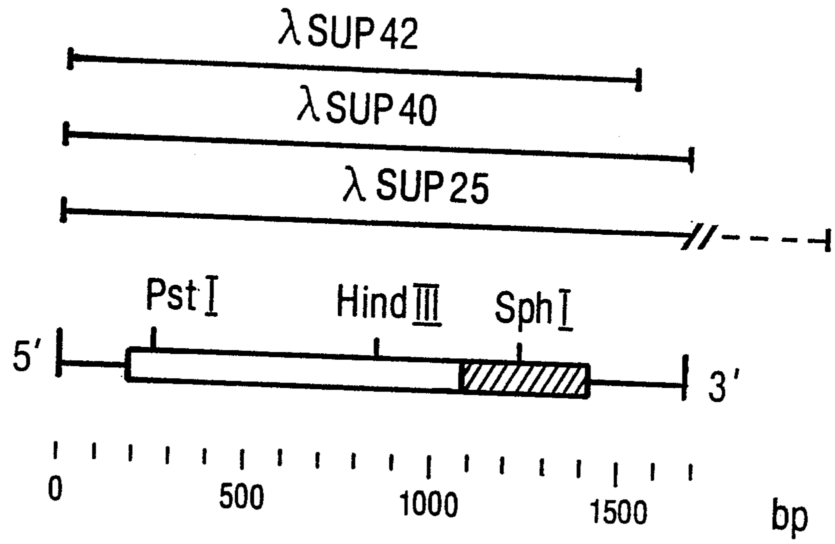

0268561

## Figure 2d : Nucleotide and amino acid sequences for the human G-TsF precursor

```
        10         20         30         40         50         60         70
CAAGCAGGAT ACGTTTTTCT GTTGGGCATT GACTAGATTG TTTGCAAAAG TTTCGCATCA AAAACAAACA

        80         90        100        110        120        130        140
ACAACAACAA AAAACCAAAC AACTCTCCTT GATCTATACT TTGAGAATTG TTGATTTCTT TTTTTTTATT

       150        160        170        180                         196
CTGACTTTTA AAAACAACTT TTTTTCCAC TTTTTTAAAAA ATG CAC TAC TGT GTG CTG AGC GCT
                                            MET His Tyr Cys Val Leu Ser Ala

    211             226             241             256
TTT CTG ATC CTG CAT CTG GTC ACG GTC GCG CTC AGC CTG TCT ACC TGC AGC ACA CTC
Phe Leu Ile Leu His Leu Val Thr Val Ala Leu Ser Leu Ser Thr Cys Ser Thr Leu

        271             286             301             316
GAT ATG GAC CAG TTC ATG CGC AAG AGG ATC GAG GCG ATC CGC GGG CAG ATC CTG AGC
Asp Met Asp Gln Phe Met Arg Lys Arg Ile Glu Ala Ile Arg Gly Gln Ile Leu Ser

            331             346             361             376
AAG CTG AAG CTC ACC AGT CCC CCA GAA GAC TAT CCT GAG CCC GAG GAA GTC CCC CCG
Lys Leu Lys Leu Thr Ser Pro Pro Glu Asp Tyr Pro Glu Pro Glu Glu Val Pro Pro

                391             406             421
GAG GTG ATT TCC ATC TAC AAC AGC ACC AGG GAC TTG CTC CAG GAG AAG GCG AGC CGG
Glu Val Ile Ser Ile Tyr Asn Ser Thr Arg Asp Leu Leu Gln Glu Lys Ala Ser Arg

436             451             466             481
AGG GCG GCC GCC TGC GAG CGC GAG AGG AGC GAC GAA GAG TAC TAC GCC AAG GAG GTT
Arg Ala Ala Ala Cys Glu Arg Glu Arg Ser Asp Glu Glu Tyr Tyr Ala Lys Glu Val

    496             511             526             541
TAC AAA ATA GAC ATG CCG CCC TTC TTC CCC TCC GAA AAT GCC ATC CCG CCC ACT TTC
Tyr Lys Ile Asp Met Pro Pro Phe Phe Pro Ser Glu Asn Ala Ile Pro Pro Thr Phe

        556             571             586             601
TAC AGA CCC TAC TTC AGA ATT GTT CGA TTT GAC GTC TCA GCA ATG GAG AAG AAT GCT
Tyr Arg Pro Tyr Phe Arg Ile Val Arg Phe Asp Val Ser Ala Met Glu Lys Asn Ala

            616             631             646             661
TCC AAT TTG GTG AAA GCA GAG TTC AGA GTC TTT CGT TTG CAG AAC CCA AAA GCC AGA
Ser Asn Leu Val Lys Ala Glu Phe Arg Val Phe Arg Leu Gln Asn Pro Lys Ala Arg

                676             691             706
GTG CCT GAA CAA CGG ATT GAG CTA TAT CAG ATT CTC AAG TCC AAA GAT TTA ACA TCT
Val Pro Glu Gln Arg Ile Glu Leu Tyr Gln Ile Leu Lys Ser Lys Asp Leu Thr Ser

721             736             751             766
CCA ACC CAG CGC TAC ATC GAC AGC AAA GTT GTG AAA ACA AGA GCA GAA GGC GAA TGG
Pro Thr Gln Arg Tyr Ile Asp Ser Lys Val Val Lys Thr Arg Ala Glu Gly Glu Trp

    781             796             811             826
CTC TCC TTC GAT GTA ACT GAT GCT GTT CAT GAA TGG CTT CAC CAT AAA GAC AGG AAC
Leu Ser Phe Asp Val Thr Asp Ala Val His Glu Trp Leu His His Lys Asp Arg Asn

        841             856             871             886
CTG GGA TTT AAA ATA AGC TTA CAC TGT CCC TGC TGC ACT TTT GTA CCA TCT AAT AAT
Leu Gly Phe Lys Ile Ser Leu His Cys Pro Cys Cys Thr Phe Val Pro Ser Asn Asn

            901             916             931             946
TAC ATC ATC CCA AAT AAA AGT GAA GAA CTA GAA GCA AGA TTT GCA GGT ATT GAT GGC
Tyr Ile Ile Pro Asn Lys Ser Glu Glu Leu Glu Ala Arg Phe Ala Gly Ile Asp Gly

                961             976             991
ACC TCC ACA TAT ACC AGT GGT GAT CAG AAA ACT ATA AAG TCC ACT AGG AAA AAA AAC
Thr Ser Thr Tyr Thr Ser Gly Asp Gln Lys Thr Ile Lys Ser Thr Arg Lys Lys Asn

1006            1021            1036            1051
AGT GGG AAG ACC CCA CAT CTC CTG CTA ATG TTA TTG CCC TCC TAC AGA CTT GAG TCA
Ser Gly Lys Thr Pro His Leu Leu Leu Met Leu Leu Pro Ser Tyr Arg Leu Glu Ser

    1066            1081            1096            1111
CAA CAG ACC AAC CGG CGG AAG AAG CGT GCT TTG GAT GCG GCC TAT TGC TTT AGA AAT
Gln Gln Thr Asn Arg Arg Lys Lys Arg Ala Leu Asp Ala Ala Tyr Cys Phe Arg Asn

        1126            1141            1156            1171
GTG CAG GAT AAT TGC TGC CTA CGT CCA CTT TAC ATT GAT TTC AAG AGG GAT CTA GGG
Val Gln Asp Asn Cys Cys Leu Arg Pro Leu Tyr Ile Asp Phe Lys Arg Asp Leu Gly

            1186            1201            1216            1231
TGG AAA TGG ATA CAC GAA CCC AAA GGG TAC AAT GCC AAC TTC TGT GCT GGA GCA TGC
Trp Lys Trp Ile His Glu Pro Lys Gly Tyr Asn Ala Asn Phe Cys Ala Gly Ala Cys

                1246            1261            1276
CCG TAT TTA TGG AGT TCA GAC ACT CAG CAC AGC AGG GTC CTG AGC TTA TAT AAT ACC
Pro Tyr Leu Trp Ser Ser Asp Thr Gln His Ser Arg Val Leu Ser Leu Tyr Asn Thr

1291            1306            1321            1336
ATA AAT CCA GAA GCA TCT GCT TCT CCT TGC TGC GTG TCC CAA GAT TTA GAA CCT CTA
Ile Asn Pro Glu Ala Ser Ala Ser Pro Cys Cys Val Ser Gln Asp Leu Glu Pro Leu

    1351            1366            1381            1396
ACC ATT CTC TAC TAC ATT GGC AAA ACA CCC AAG ATT GAA CAG CTT TCT AAT ATG ATT
Thr Ile Leu Tyr Tyr Ile Gly Lys Thr Pro Lys Ile Glu Gln Leu Ser Asn Met Ile

        1411            1426       1436       1446       1456       1466
GTA AAG TCT TGC AAA TGC AGC TAA AATTCTTGGA AAAGTGGCAA GACCAAAATG ACAATGATGA
Val Lys Ser Cys Lys Cys Ser

      1476       1486       1496       1506       1516       1526       1536
TGATAATGAT GATGACGACG ACAACGATGA TGCTTGTAAC AAGAAAACAT AAGAGAGCCT TGGTTCATCA

      1546       1556       1566       1576       1586       1596       1606
GTGTTAAAAA ATTTTTGAAA AGGCGGTACT AGTTCAGACA CTTTGGAAGT TTGTGTTCTG TTTGTTAAAA

      1616       1626       1636       1646       1656       1666       1676
CTGGCATCTG ACACAAAAAA AGTTGAAGGC CTTATTCTAC ATTTCACCTA CTTTGTAAGT GAGAGAGACA

      1686
AGAAGCAAAT TTTTTTAAA
```

0268561

Figure 2e: Comparison of the amino acid sequences of G-TsF and TGF-β1 precursors

```
                        11        21        31        41        51        61    66        76        86
G-TsF         1 MHYCVLSAFLILHLVTVALSLSTCSTLDMDQFMRKRIEAIRGQILSKLKLTSPPEDYPEPEEVPP-----EVISIYNSTRDLLQEKASRRA
                          *  *      *  **** * **     *************** * ***        ****      *  ******        *
TGF-β1      1 MPPSGLRLLPLLLPLLWLLVLTPGPPAAGLSTCKTIDMELVKRKRIEAIRGQILSKLRLASPP----SQGEVPPGPLPEAVLALYNSTRD--------RV
                   10        20        30        40        50        60    66        76        86       88

     96     103       113       123       133             149       159       169       179
AACERERSDE---EYYAKEVYKIDMPPFFPSENAIPPTFYRPYFRIVRFDVSAMEKNA----SNLVKAEFRVFRLQNPKARVPEQRIELYQILKSKDLTS
*    *    *    ******    *        * *    *          *    *      *  ** *  *    *    **  ****
AGESAEPEPEPEADYYAKEVTRVLM---VETHNEIYDKFKQSTHSIYMFFNTSELREAVPEPVLLSRAELRLLRRLKLK---VEQHVELYQ-----KYSN
     98     108       115       125       135       145       155                     172      177

     189       199       209       219       229       239       249       259       269       279
PTQRYIDSKVVKTRAEGEWLSFDVTDAVHEWLHHKDRNLGFKISLHCPCCTFVPSNNYIIPNKSEELEARFAGIDGTSTYTSGDQKTIKSTRKKNSGKTP
   **           ********  *  **       **  *  ** *                           * *  *    **  **             *
NSWRYLSNRLLAPSDSPEWLSFDVTGVVRQWLSRGGEIEGFRLSAHCSC--------------DSRDNTLQVDINGFTTGRRGDLATI------HGMNRP
     187       197       207       217                         233       243                   257

     289       299       309       319       329       339       349       359       369       379
HLLLMLLPSYRLESQQTNRRKKRALDAAYCFRNVQDNCCLRPLYIDFKRDLGWKWIHEPKGYNANFCAGACPYLWSSDTQHSRVLSLYNTINPEASASPC
 ****  *  *    *    ****  ***      *** * *****  ************** **** * *** ** *** * ** ***   ** *** **
FLLLMATPLERAQHLQ-SSRHRRALDTNYCFSSTEKNCCVRQLYIDFRKDLGWKWIHEPKGYHANFCLGPCPYIWSLDTQYSKVLALYNQHNPGASAAPC
     267       276       286       296       306       316       326       336       346       356

     389       399       409
CVSQDLEPLTILYYIGKTPKIEQLSNMIVKSCKCS   414
** * **** * ** *   ** ******** *****
CVPQALEPLPIVYYVGRKPKVEQLSNMIVRSCKCS   391
     366       376       386
```

Figure 3a : CHO- and COS-cells expression vector

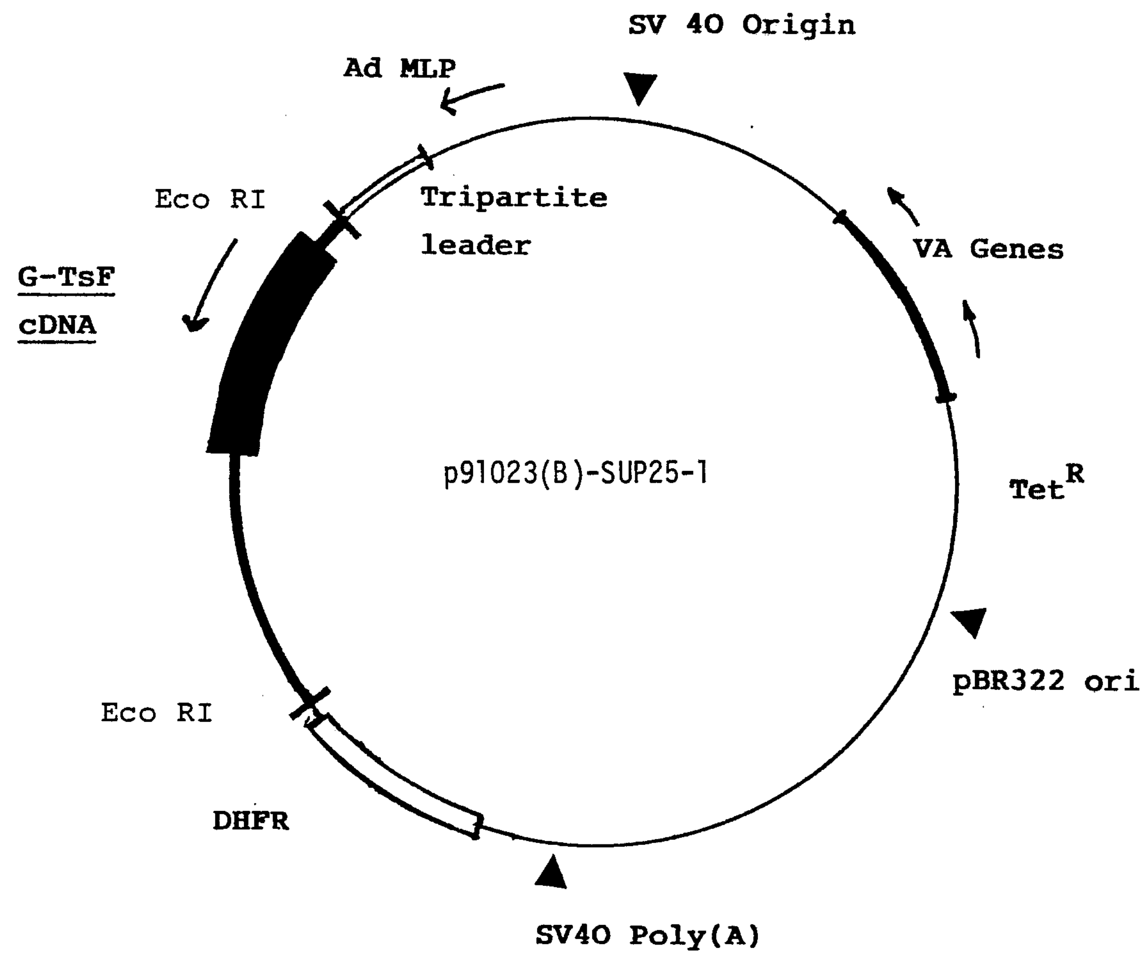

Figure 3b : Expression plasmid for CHO- and COS- cells with SUP40 cDNA

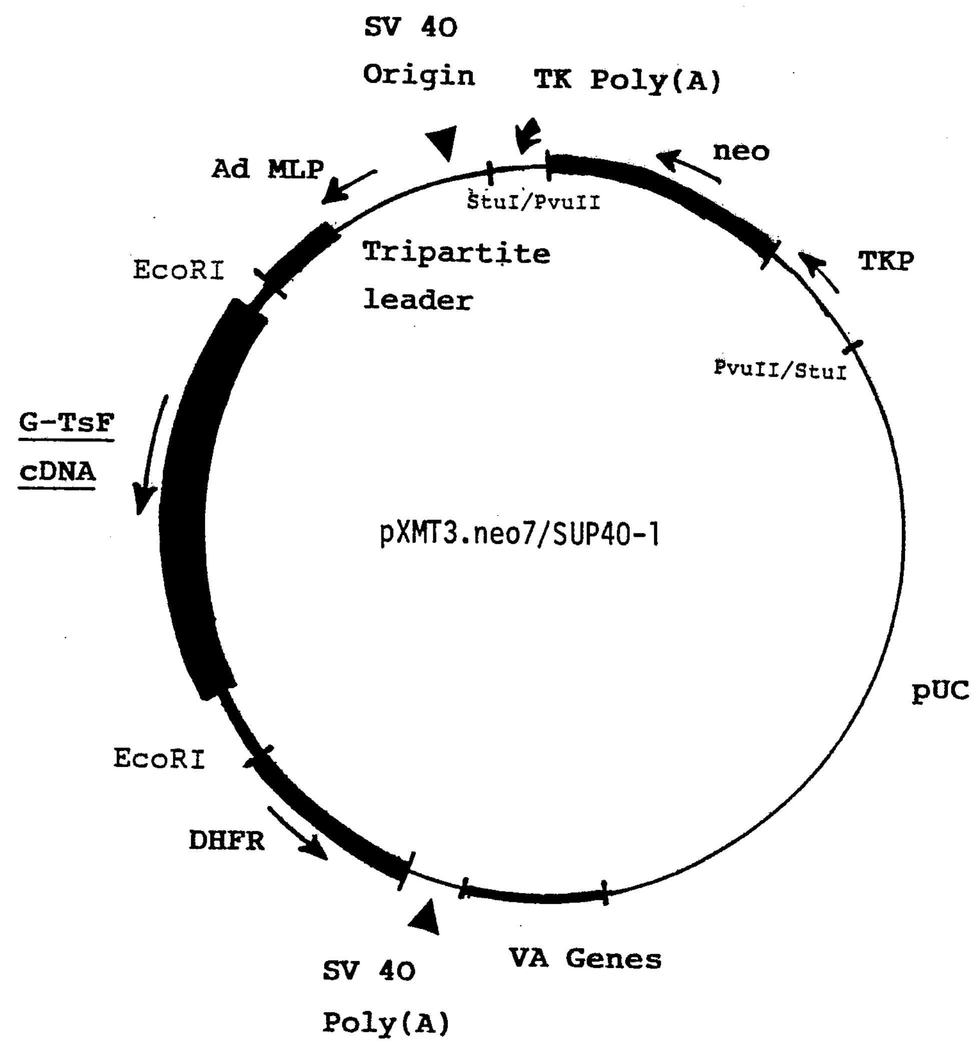

Figure 4 : Final purification step for G-TsF from 308 glioblastoma cells from 1 l of glioblastoma supernatant on Pro-RPCTM with trifluoroacetic acid/isopropanol

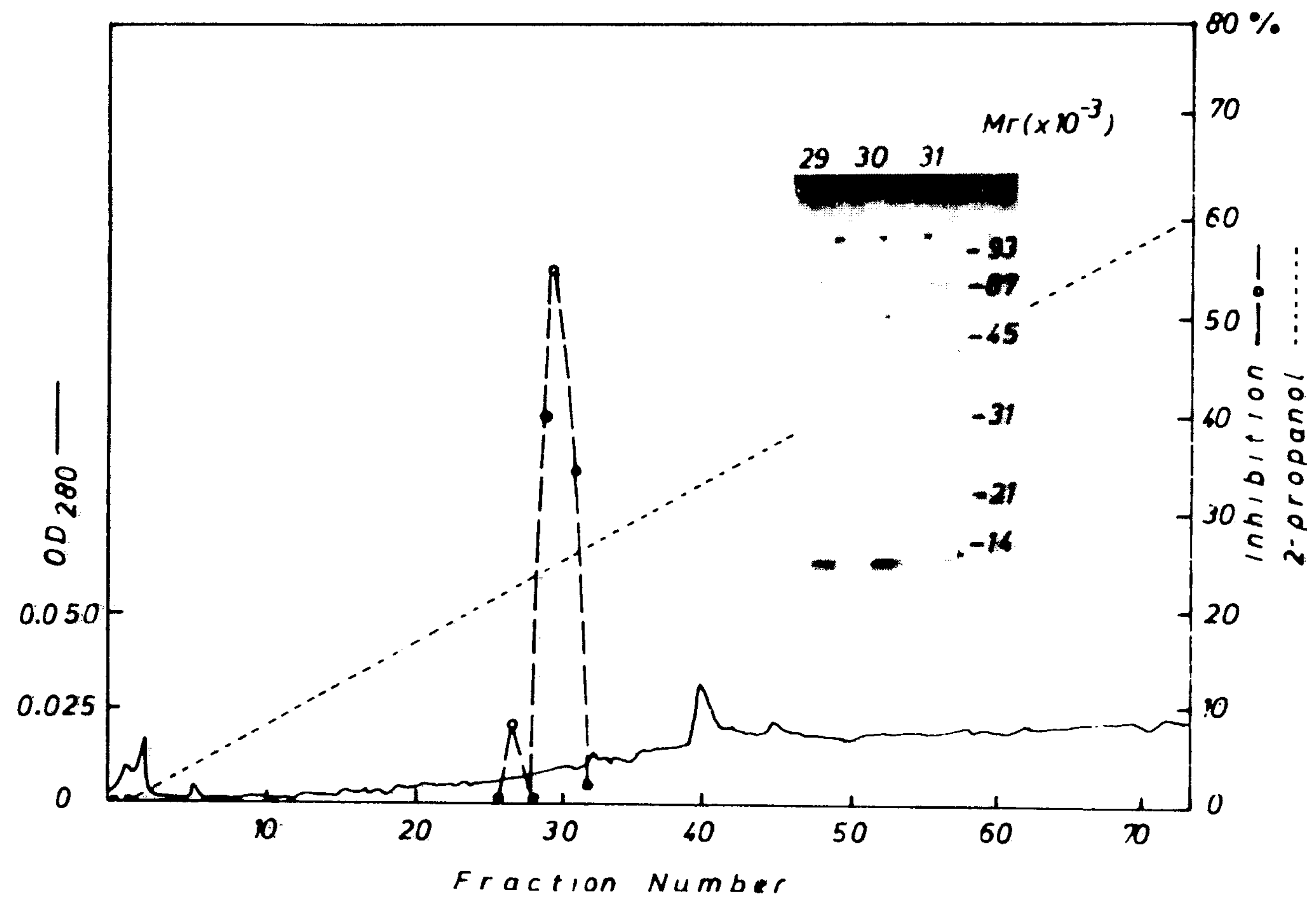

Figure 5: Activity of pure human G-TsF

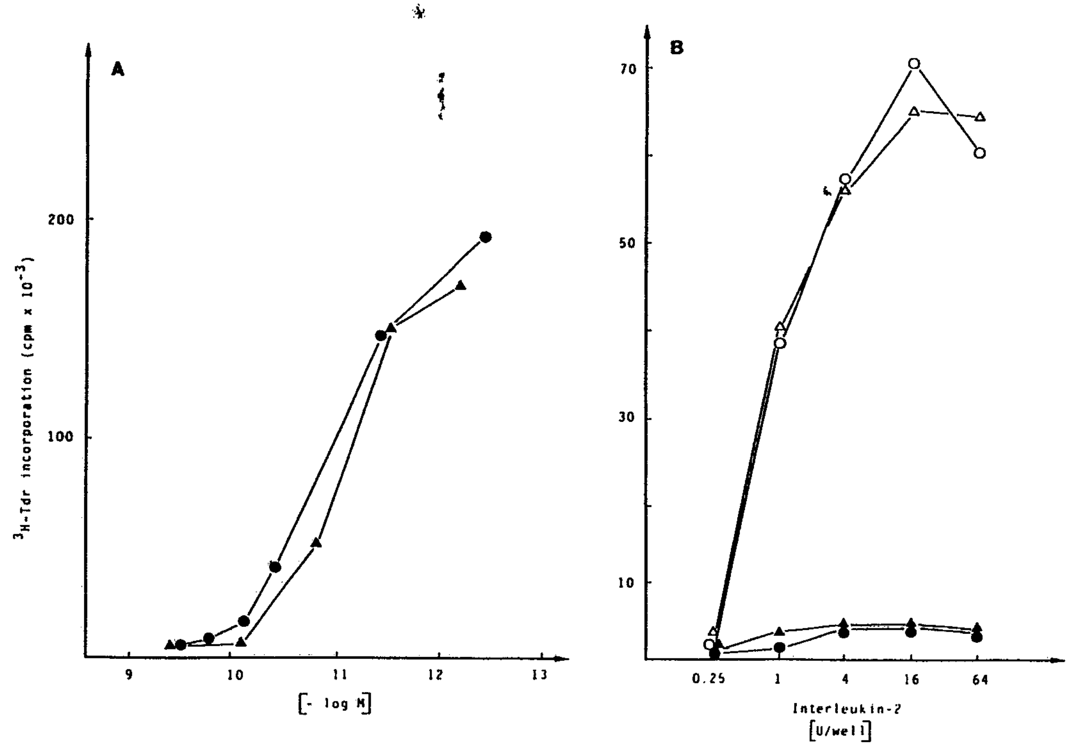

Figure 6 : Final purification of G-TsF from 10 l of glioblastoma supernatant on Pro-RPC™ with TFA/isopropanol

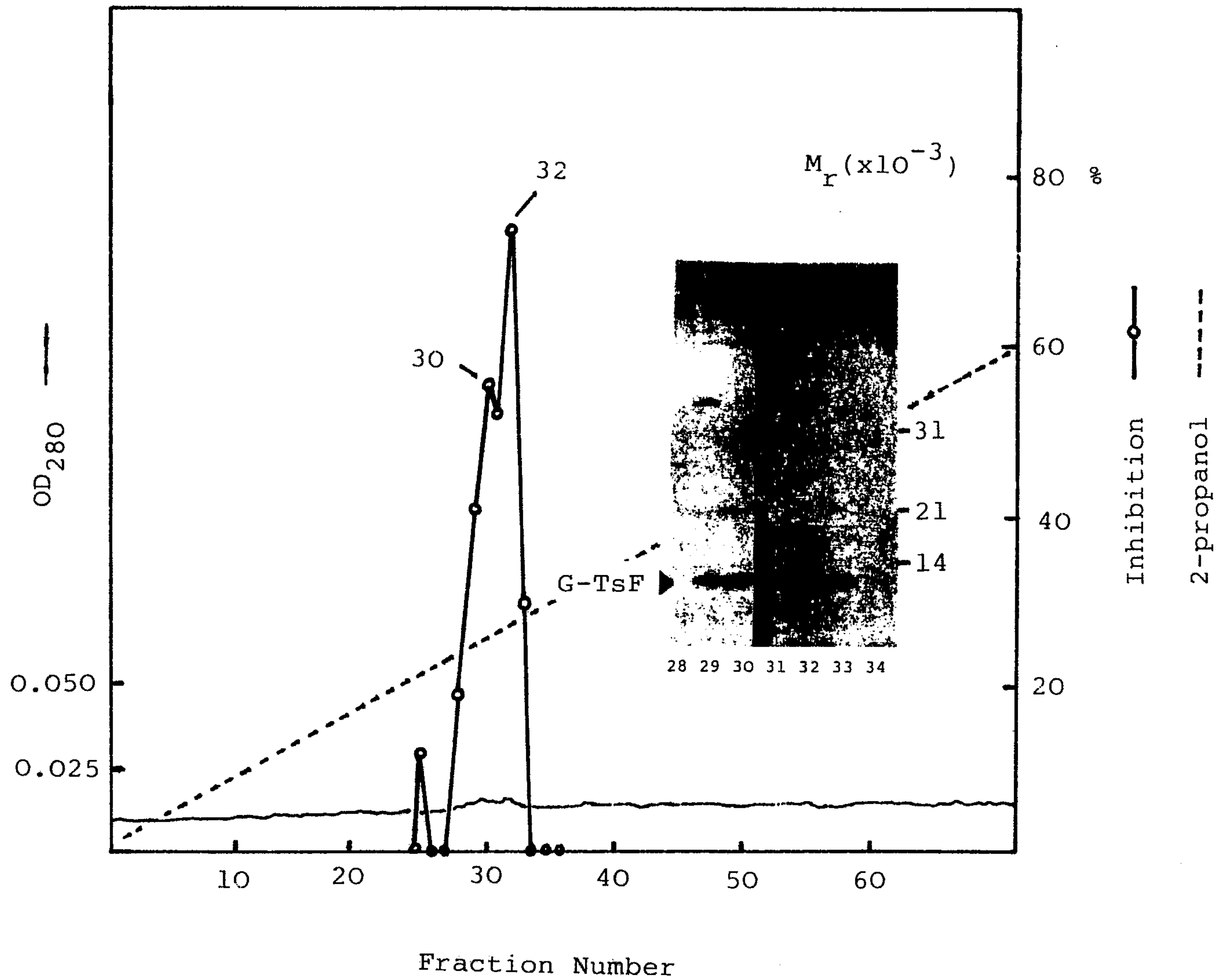

Figure 7a: Plasmid for expression of G-TsF in E. coli based on the lambda promoter

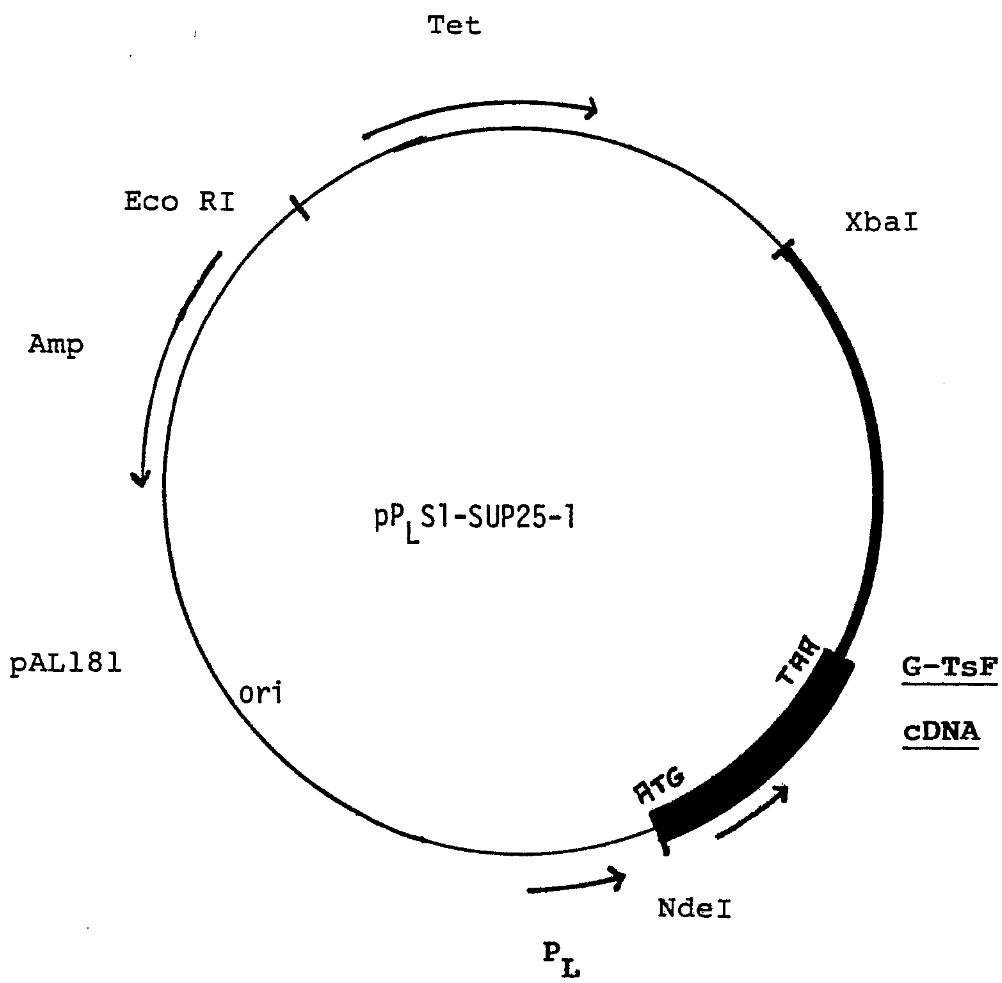

0268561

Figure 7b: Construction of the expression plasmid $pP_LS1$-SUP25-1

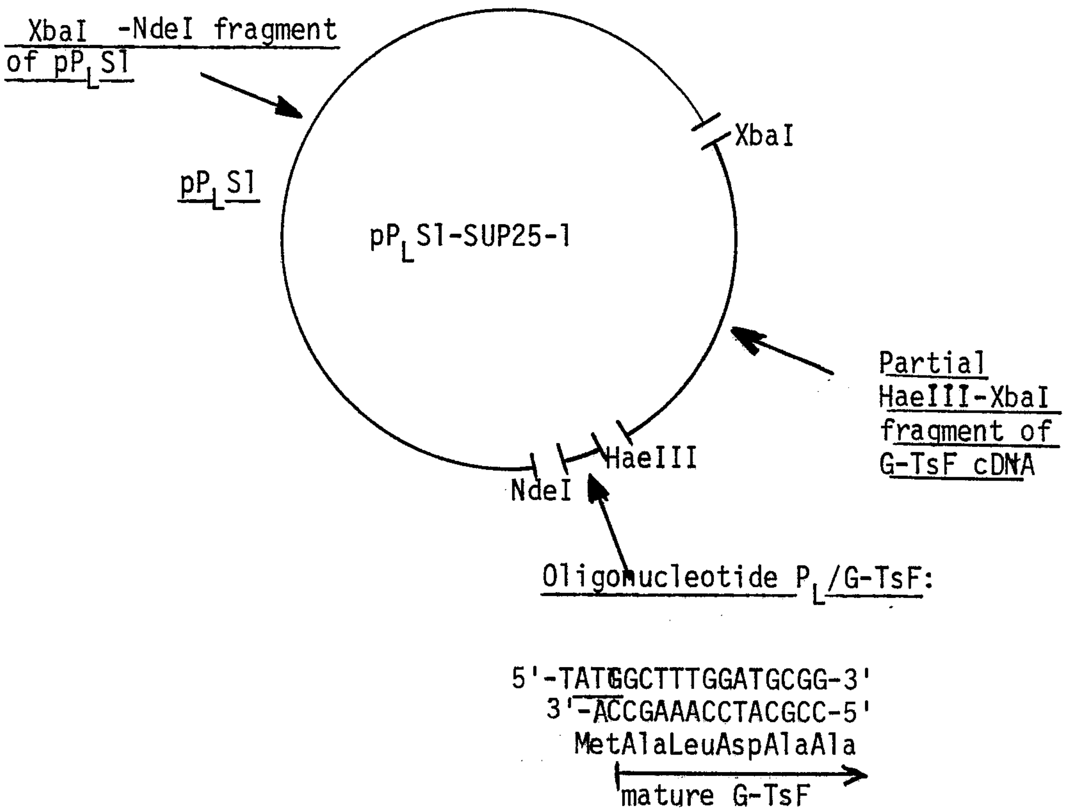

Figure 8 : Expression and purification of recombinant G-TsF from E. coli

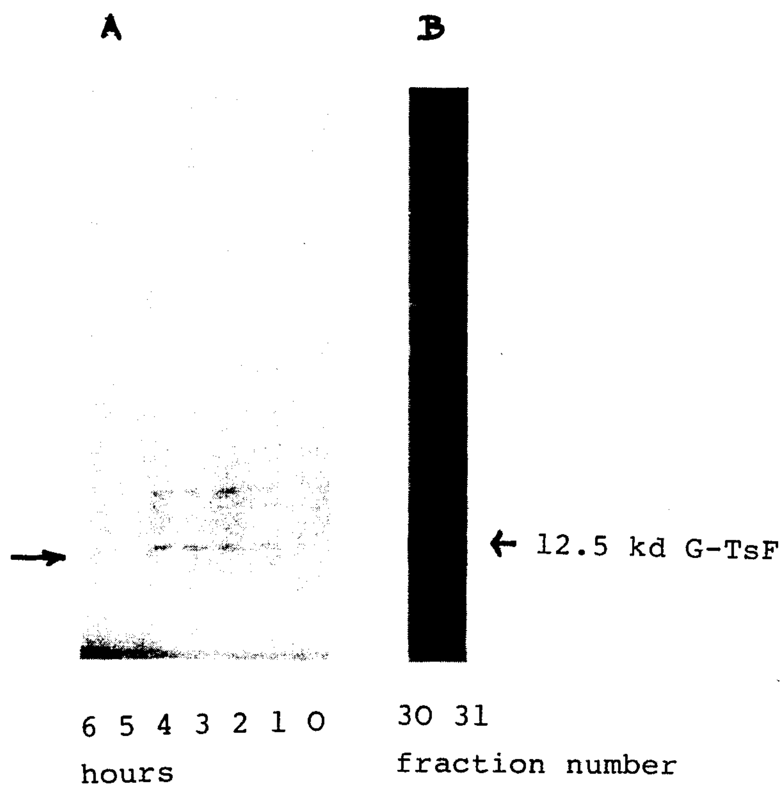

Figure 9: Inhibition of thymocyte proliferation by recombinant G-TsF from E. coli

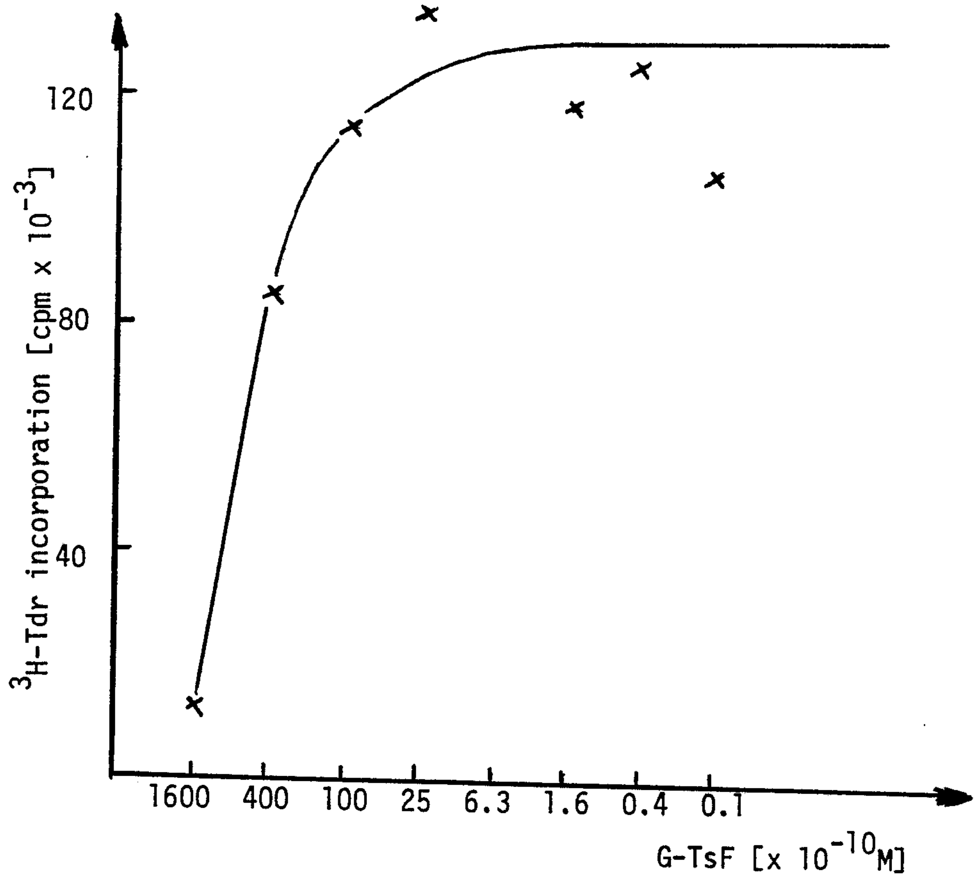

0268561

Figure 10 : Demonstration of expression of recombinant G-TsF by CHO cells

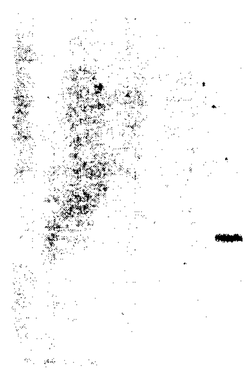

← 12.5 kd
G-TsF

1 2 3

Figure 11 : Inhibition of --- chymocyte proliferation ---- with recombinant G-TsF from CHO cells